# EUROPEAN PATENT APPLICATION

(11) **EP 2 085 084 A1**
(43) Date of publication of application: **05.08.2009**
(21) Application number: 08150776.6
(22) Date of filing: 29.01.2008
(51) Int. Cl.: A61K 31/397, A61P 31/04, C07D 477/00

(54) **Use of inhibitor of beta-lactamases and its combination with beta-lactam antibiotics**

(71) Applicant: LEK Pharmaceuticals d.d., 1526 Ljubljana (SI)
(72) Inventor: Prezelj, Andrej, 1000, Ljubljana (SI); Urleb, Uros, 1000, Ljubljana (SI); Vilfan, Gregr, 1000, Ljubljana (SI)
(74) Representative: Kröger, Bernd

(57) **Abstract**

The present invention relates to A pharmaceutical composition with broad-spectrum of activity against class A, class C and D enzymes comprising an antibiotic and a pharmaceutically effective amount of a compound of formula (I) 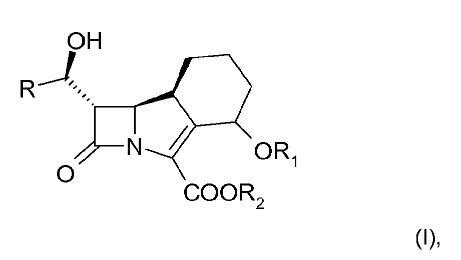 compounds of formula (I), the use of a therapeutically effective amount of one or more compounds of formula (I) as a broad-spectrum beta-lactamase inhibitor and the use of such a pharmaceutical composition for the treatment of an infection in humans or animals caused by bacteria.

## Description

### FIELD OF INVENTION

The present invention relates to the field of new antimicrobial drugs, to a synergistic pharmaceutical and veterinary compositions comprising inhibitor of beta-lactamases of formula (I) or pharmaceutically acceptable salts, esters or amides thereof in combination with an antibiotic, especially with an antibiotic that is susceptible to degradation by beta-lactamase and the use of a respective pharmaceutical composition for the treatment of an infection in humans or animals caused by a bacterium.

### BACKGROUND OF THE INVENTION

The dramatic worldwide increase in the number of bacterial strains acquiring resistance to the beta-lactam antibiotics has become one of the most important threats to modern health care. The dissemination of existing beta-lactamases and the evolution of new enzymes with extended substrate profiles are the most common and often the most efficient mechanism of bacterial resistance to beta-lactam antibiotics. Currently the beta-lactamase super-family has more than 550 members, many of which differ only by a single amino acid. Based on amino-acid sequence similarities, beta-lactamases have been broadly grouped into four molecular classes, A, B, C and D. [Bush K; et al; Antimicrob. Agents Chemother. 1995, 39 (6): 1211-1233; Thomson KS; et al; Microbes and Infections 2000, 2: 1225-1235].

The bacterial beta-lactamase enzymes hydrolyze antibiotics of beta-lactam family, e.g. penicillins, cephalosporins, monobactams, carbapenems, to inactive products by hydrolyzing the beta-lactam bond. One counter-strategy is to co-administer inhibitor of beta-lactamases such as clavulanate, sulbactam, or tazobactam, that have been successfully used in combinations against bacteria producing the ubiquitous and prevalent TEM-1 and SHV-1 class A beta-lactamases. However, little or no activity against class C and B enzymes was observed. In addition, bacterial susceptibility to such combinations has recently been challenged by the spontaneous appearance of new beta-lactamases of the TEM family, which are resistant to the mechanism-based inactivators in the market. Any organism with an inducible AmpC beta-lactamase (class C) can segregate derepressed mutants, and any TEM, SHV or CTX-M producer can segregate ESBL (extended spectrum beta-lactamase) variants. [Livermore, DM. J. Antimicrob. Chemother. 1998, 41 (D), 25-41; Livermore, DM. Clinical Microbiology Reviews 1995, 8 (4), 557-584; Helfand MS; et al; Curr. Opin. Pharmacol. 2005, 5: 452-458].

Attempts to address the above mentioned problems through the development of inhibitor of beta-lactamases had only limited success in the past. However, a detailed knowledge of binding mechanism and interactions of known beta-lactams should facilitate the design of novel beta-lactam that will bypass this defense mechanism.

Alkylidene penems and 2-beta-substituted penam sulphones, oxapenems, cephalosporin-derived compounds, cyclic acyl phosphonates, and non-beta-lactam compounds are currently under investigation as potential inhibitors of beta-lactamases, but their clinical applications are not yet available [Buynak JD. Curr. Med. Chem. 2004, 11, 1951-1964; Bonnefoy A et al, J. Am. Chem. Soc. 2004, 54, 410-417; Weiss WJ et al; Antimicrob. Agents Chemother. 2004, 48, 4589-4596, Phillips OA at al; J. Antibiot. 1997, 50, 350-356; Jamieson CE et al; Antimicrob. Agents Chemother. 2003, 47, 1652-1657].

An alternative strategy would be discovery of new antibiotics that are stable to clinically relevant beta-lactamases. Several beta-lactam antibiotics have been designed by introducing bulky substituents that sterically hinder binding to the beta-lactamases.

Sanfetrinem cilexetil (GV-118819) is an orally absorbed prodrug ester of sanfetrinem sodium (GV-104326), a highly potent broad-spectrum tricyclic beta-lactam antibiotic (trinem) which is active *in vitro* and *in vivo* against a wide range of Gram-positive, Gram-negative and anaerobic bacteria, except *Pseudomonas aeruginosa* and methicillin-resistant *Staphylococcus aureus.* Its activity was superior to several cefalosporins against *Escherichia coli, Klebsiella pneumoniae, Klebsiella oxytoca, Citrobacter diversus, Proteus mirabilis, Proteus vulgaris, Morganella morganii, Providencia rettgeri, Haemophilus* spp., and *Moraxella catarrhalis.* The compound is active against *Enterococcus faecalis, Enterococcus faecium, S. aureus,* streptococci, *Rhodococcus-*like species, and anaerobes. [SK Spangler, et al, Antimicrob. Agents Chemother. 1997, 41, 1, 148-155].

Sanfetrinem shared the stability of imipenem and meropenem to AmpC and ESBLs. The researchers concluded that sanfetrinem has beta-lactamase interactions similar to those of the available carbapenems except that it is a weaker inducer of AmpC types, with some tendency to select derepressed mutants, unlike imipenem and meropenem. The *k*_{cat} values of AmpC for cefpodoxime (8 s⁻¹) and cefixime (10 s⁻¹) were higher than that for sanfetrinem (0.00033 s⁻¹), underlining the enzyme's greater ability to confer resistance to the cephalosporins than to the trinem. [GS Babini, et al, Antimicrob. Agents Chemother. 1998, 42, 5, 1168-1175].

The safety and tolerability of single and multiple doses of sanfetrinem were evaluated in healthy subjects. Sanfetrinem was administered in single doses of 0.25-4 g/day, i.v. or repeated doses of 1.5-3 g/day, i.v., and orally in single doses of 0.25-2 g/day, i.v. or repeated doses of 0.25-1 g/day, i.v. No changes were noted in blood pressure, pulse, peak expiratory flow rate and ECGs for both single and repeated doses. Clinical chemistry, hematology, coagulation and urinalysis safety screens also showed no serious results, thus demonstrating sanfetrinem's safety and good tolerability. [J Ngo, et al, Drugs Future. 1996, 21, 12, 1238-1245].

Therefore, one point of interest according to the present invention is the improvement of the stability of enzyme-inhibitor complexes and the design of efficient compounds with high acylation and low deacylation rates that are resistant to inactivation by beta-lactamases. Another subject of the present invention is to provide new pharmaceutical compositions that show a potency and spectrum for the most prevalent clinically relevant resistant strains.

It has been found that inhibitor of beta-lactamases of formula (I) and also salts, esters or amides thereof, used as inhibitor of beta-lactamases are suitable to combat emerging bacterial resistance in class A, class C and class D beta-lactamases and provide a vital addition to the hospital antibiotic armory, in particular in a pharmaceutical composition additionally comprising an antibiotic.

Even more, due to the antibiotic activity of inhibitor of beta-lactamases of formula (I) alone the additive extension of the beta-lactam antibiotic spectrum is deemed beneficial to provide broader coverage of clinically important pathogens.

Also the mutation frequency is expected to be lower due to the combination product.

Unexpectedly, it has been found that a combination of said inhibitor of beta-lactamases of formula (I) and an antibiotic, in particular a beta-lactam antibiotic show an advanced synergistic antimicrobial effect. The activity of the combination is considered synergistic as the measured effect significantly exceeded any additive effects of the two drugs.

### SUMMARY OF THE INVENTION

Thus, the present invention is directed towards a pharmaceutical composition with broad-spectrum of activity against class A, class C and D enzymes comprising an antibiotic and a pharmaceutically effective amount of a compound of formula (I) 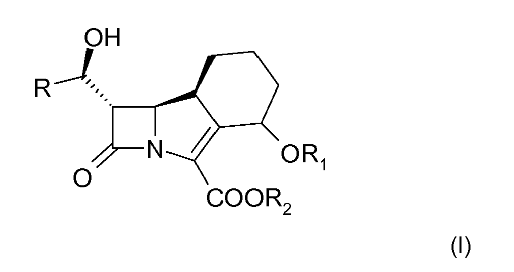 wherein
R represents a hydrogen atom or a saturated alkyl chain with 1 to 20 carbon atoms and the saturated alkyl chain may be straight (such as methyl, ethyl, n-propyl, n-butyl) or branched in any position (such as isopropyl, s-butyl, isobutyl, isoamyl, tert-butyl);
R¹ represents:
- a hydrogen atom,
- a saturated alkyl chain with 1 to 20 carbon atoms and the saturated alkyl chain may be straight (such as methyl, ethyl, n-propyl, n-butyl) or branched in any position (such as isopropyl, s-butyl, isobutyl, isoamyl, tert-butyl) and each chain member may be mono or disubstituted with substituents such as halo (such as fluoromethyl, tritluoromethyl, 2-chloroethyl), hydroxy (such as hydroxymethyl, 2-hydroxyethyl), (C_{I}-C₄)-alkyloxy (such as methoxymethyl, 2-methoxyethyl), mercapto and (C_{I}-C₄)-alkylmercapto (such as mercaptomethyl, 2-methylmercaptoethyl), (C_{I}-C₄)-alkanesulfonyl (such as methanesulfonylmethyl), amino, (C_{I}-C₄)-alkylamino and di(C_{I}-C₄)-alkylamino (such as 2-aminoethyl, 2-methylaminoethyl, 2-dimethylaminoethyl), alkyleneamino (such as 2-(1-piperidinyl)ethyl, 1-pyrrolidinylmethyl), guanidino (such as guanidinomethyl), unsubstituted N¹-mono, N³-mono, N¹,N³-di and N³,N³-di-(C_{I}-C₄)-formamidino (such as iminomethylaminomethyl, 2-(dimethylaminomethyleneamino)ethyl), aromatic or heteroaromatic five- or six-membered ring (such as phenyl, furyl, 2-pyridyl), (C_{I}-C₄)-alkyloxycarbonyl (such as carbethoxymethyl), cyano (such as 2-cyanoethyl), oxo (such as acetyl, propionyl, 2-oxopropyl),
- an unsaturated alkyl chain with 1 to 20 carbon atoms and the unsaturated alkyl chain may be straight with double bonds or triple bonds (such as vinyl, propenyl, allyl, ethinyl, propargyl) or branched in any position with double bonds or triple bonds (such as 2-propenyl) and each chain member may be mono or disubstituted with substituents such as halo, hydroxy, (C_{I}-C₄)-alkyloxy, thio and (C_{I}-C₄)-alkylthio, (C_{I}-C₄)-alkanesulfonyl, amino, (C_{I}-C₄)-alkylamino and di-(C_{I}-C₄)-alkylamino, aromatic or heteroaromatic five- or six-membered ring (such as phenyl, furyl, 2-pyridyl), (C_{I}-C₄)-alkyloxycarbonyl, cyano, oxo,
- a saturated or partly unsaturated cycloalkyl radical with 3 to 7 members (such as radicals from cyclopropyl to cycloheptyl, cyclohex-I-enyl) and the ring may comprise one or more oxygen, sulfur or nitrogen atoms (such as 2-tetrahydrofuranyl, 1-piperidinyl, 1-pyrrolidinyl) and each ring member may be mono or disubstituted with substituents such as halo, hydroxy, (C_{I}-C₄)-alkyloxy, thio and (C_{I}-C₄)-alkylthio), (C_{I}-C₄)-alkanesulfonyl, amino, (C_{I}-C₄)-alkylamino and di-(C_{I}-C₄)-alkylamino, (C_{I}-C₄)-alkyloxycarbonyl, cyano. oxo,
- an aromatic or heteroaromatic five- or six-membered ring (such as furyl, pyranyl),
- an alkanoyl (such as formyl, acetyl, benzoyl, ethoxycarbonyl, allyloxycarbonyl, pivaloyl), an alkenoyl (such as allylcarbonyl), an aroyl (such as p-nitrobenzoyl), an alkoxycarbonyl (such as t-buthoxycarbonyl), a haloalkoxy-carbonyl (such as 2,2,2-trichloroethoxycarbonyl, or 1,1,1-trichloro-2-methyl-2propoxycarbonyl), an aralkyloxycarbonyl (such as benzyloxycarbonyl or p-nitrobenzyloxycarbonyl), an alkenyloxycarbonyl (such as allyloxycarbonyl) mono, di or tri-(C_{I}-C₄)-alkylsilyl (such as trimethylsilyl, tert-butyldimethylsilyl) group,
- a (C_{I}-C₄)-alkanesulfonyl group (such as methanesulfonyl, ethanesulfonyl), a (C_{I}-C₄)-alkenesulfonyl group (such as allylsulfonyl), arylsulfonyl group (such as p-nitrobenzylsulfonyl);
R² represents:
- a hydrogen atom,
- an alkali metal,
- an earth alkali metal,
- the ammonium ion or a protonated form of mono, di or trisubstituted acyclic or cyclic aliphatic amine or a protonated form of some other nitrogen base,
- the quaternized ammonium ion,
- a (C_{I}-C₂₀)-alkyl (such as methylethyl, tert-butyl), (C_{I}-C₂₀)-alkenyl (such as allyl), substituted alkyl (such as (C_{I}-C₄)-alkoxyalkyl, (C_{I}-C₄)-alkylthioalkyl, phenetyl, 2,2,2-trichloroethyl, 2-oxo-5-methyl-1,3-dioxolene-4-yl)methyl, benzyl, p-methoxybenzyl, p-nitrobenzyl, o-nitrobenzyl, bis(methoxyphenyl)methyl, 3,4-dimethoxybenzyl, benzhydryl, trityl, 2-trimethylsilylethyl), substituted silyl (such as trimethylsilyl, tert-butyldimethylsilyl), phthalidyl etc., or
- a radical which may be presented in a following form 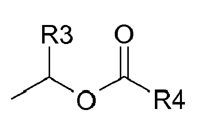 wherein R³ represents hydrogen or a lower alkyl with 1 to 4 carbon atoms, and
   R⁴ represents hydrogen, alky, cycloalkyl, alkoxy, cycloalkoxy, cycloalkylalkyl, alkenyloxy, phenyl, 2-morpholinoethyl,
or a salt, ester or amide derivate of the compound of formula (I).

A prefered process for the preparation of compounds of formula (I) is described within WO 92/03437 A. (WO 92/03437 - EP 0 495 953 B1 - Glaxo - Titel: 10 (1-Hydroxyethyl) -11-oxo-1-azatricyclo[7.2.0.0 (3,8)]undec-2-ene-2-carboxylic acid esters and a process for preparing it.). Compounds of the formula (I) are also disclosed in WO 94/21637, WO 94/21638 and WO 98/27094.

The present invention is also directed towards a synergistic pharmaceutical composition comprising a compound of formula (I) as disclosed above and a beta-lactam antibiotic.

These pharmaceutical compositions according to the present invention may additionally comprise a pharmaceutically acceptable carrier and/or a pharmaceutically acceptable excipient. These pharmaceutical compositions are particularly suitable for treatment of an infection in humans or animals caused by a bacterium, especially a bacterium that produces a significant amount of beta-lactamase.

The present invention is also directed to the use of a therapeutically effective amount of one or more compounds of formula (I) as defined above or a salt, ester or amide derivative thereof as a broad-spectrum beta-lactamase inhibitor, in particular wherein the beta-lactamase inhibitor is a beta-lactamase inhibitor of class A, C and D.

The present invention is also directed to the use of a pharmaceutical composition as disclosed above for the treatment of an infection in humans or animals caused by bacteria.

Finally, the present invention is also directed to the use of a therapeutically effective amount of the composition according to the present invention and at least one pharmaceutically acceptable excipient for the preparation of a medicament for treating an infection caused by bacteria, preferably wherein said medicament is to be administered to a patient in need thereof.

Additionally, the present invention is directed to a method of treating an infection in humans or animals caused by bacteria comprising administering to a patient in need of such treating a therapeutically effective amount of the composition according to the present invention and at least one pharmaceutically acceptable excipient.

### DETAILED DESCRIPTION OF THE INVENTION

The present invention relates to a pharmaceutical composition with broad-spectrum of activity against class A, class C and D enzymes comprising an antibiotic and a pharmaceutically effective amount of a compound of formula (I) 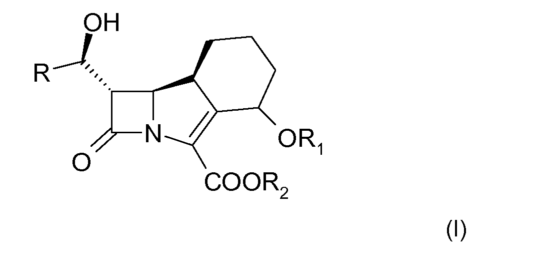 wherein
R represents a hydrogen atom or a saturated alkyl chain with 1 to 20 carbon atoms and the saturated alkyl chain may be straight (such as methyl, ethyl, n-propyl, n-butyl) or branched in any position (such as isopropyl, s-butyl, isobutyl, isoamyl, tert-butyl);
R¹ represents:
- a hydrogen atom,
- a saturated alkyl chain with 1 to 20 carbon atoms and the saturated alkyl chain may be straight (such as methyl, ethyl, n-propyl, n-butyl) or branched in any position (such as isopropyl, s-butyl, isobutyl, isoamyl, tert-butyl) and each chain member may be mono or disubstituted with substituents such as halo (such as fluoromethyl, tritluoromethyl, 2-chloroethyl), hydroxy (such as hydroxymethyl, 2-hydroxyethyl), (C_{I}-C₄)-alkyloxy (such as methoxymethyl, 2-methoxyethyl), mercapto and (C_{I}-C₄)-alkylmercapto (such as mercaptomethyl, 2-methylmercaptoethyl), (C_{I}-C₄)-alkanesulfonyl (such as methanesulfonylmethyl), amino, (C_{I}-C₄)-alkylamino and di(C_{I}-C₄)-alkylamino (such as 2-aminoethyl, 2-methylaminoethyl, 2-dimethylaminoethyl), alkyleneamino (such as 2-(1-piperidinyl)ethyl, 1-pyrrolidinylmethyl), guanidino (such as guanidinomethyl), unsubstituted N¹-mono, N³-mono, N¹,N³-di and N³,N³-di-(C₁-C₄)-formamidino (such as iminomethylaminomethyl, 2-(dimethylaminomethyleneamino)ethyl), aromatic or heteroaromatic five- or six-membered ring (such as phenyl, furyl, 2-pyridyl), (C_{I}-C₄)-alkyloxycarbonyl (such as carbethoxymethyl), cyano (such as 2-cyanoethyl), oxo (such as acetyl, propionyl, 2-oxopropyl),
- an unsaturated alkyl chain with 1 to 20 carbon atoms and the unsaturated alkyl chain may be straight with double bonds or triple bonds (such as vinyl, propenyl, allyl, ethinyl, propargyl) or branched in any position with double bonds or triple bonds (such as 2-propenyl) and each chain member may be mono or disubstituted with substituents such as halo, hydroxy, (C_{I}-C₄)-alkyloxy, thio and (C_{I}-C₄)-alkylthio, (C_{I}-C₄)-alkanesulfonyl, amino, (C_{I}-C₄)-alkylamino and di-(C_{I}-C₄)-alkylamino, aromatic or heteroaromatic five- or six-membered ring (such as phenyl, furyl, 2-pyridyl), (C_{I}-C₄)-alkyloxycarbonyl, cyano, oxo,
- a saturated or partly unsaturated cycloalkyl radical with 3 to 7 members (such as radicals from cyclopropyl to cycloheptyl, cyclohex-I-enyl) and the ring may comprise one or more oxygen, sulfur or nitrogen atoms (such as 2-tetrahydrofuranyl, 1-piperidinyl, 1-pyrrolidinyl) and each ring member may be mono or disubstituted with substituents such as halo, hydroxy, (C_{I}-C₄)-alkyloxy, thio and (C_{I}-C₄)-alkylthio), (C_{I}-C₄)-alkanesulfonyl, amino, (C_{I}-C₄)-alkylamino and di-(C_{I}-C₄)-alkylamino, (C_{I}-C₄)-alkyloxycarbonyl, cyano. oxo,
- an aromatic or heteroaromatic five- or six-membered ring (such as furyl, pyranyl),
- an alkanoyl (such as formyl, acetyl, benzoyl, ethoxycarbonyl, allyloxycarbonyl, pivaloyl), an alkenoyl (such as allylcarbonyl), an aroyl (such as p-nitrobenzoyl), an alkoxycarbonyl (such as t-buthoxycarbonyl), a haloalkoxy-carbonyl (such as 2,2,2-trichloroethoxycarbonyl, or 1,1,1-trichloro-2-methyl-2propoxycarbonyl), an aralkyloxycarbonyl (such as benzyloxycarbonyl or p-nitrobenzyloxycarbonyl), an alkenyloxycarbonyl (such as allyloxycarbonyl) mono, di or tri-(C_{I}-C₄)-alkylsilyl (such as trimethylsilyl, tert-butyldimethylsilyl) group,
- a (C_{I}-C₄)-alkanesulfonyl group (such as methanesulfonyl, ethanesulfonyl), a (C_{I}-C₄)-alkenesulfonyl group (such as allylsulfonyl), arylsulfonyl group (such as p-nitrobenzylsulfonyl);
R² represents:
- a hydrogen atom,
- an alkali metal,
- an earth alkali metal,
- the ammonium ion or a protonated form of mono, di or trisubstituted acyclic or cyclic aliphatic amine or a protonated form of some other nitrogen base,
- the quaternized ammonium ion,
- a (C_{I}-C₂₀)-alkyl (such as methylethyl, tert-butyl), (C₁-C₂₀)-alkenyl (such as allyl), substituted alkyl (such as (C_{I}-C₄)-alkoxyalkyl, (C_{I}-C₄)-alkylthioalkyl, phenetyl, 2,2,2-trichloroethyl, 2-oxo-5-methyl-1,3-dioxolene-4-yl)methyl, benzyl, p-methoxybenzyl, p-nitrobenzyl, o-nitrobenzyl, bis(methoxyphenyl)methyl, 3,4-dimethoxybenzyl, benzhydryl, trityl, 2-trimethylsilylethyl), substituted silyl (such as trimethylsilyl, tert-butyldimethylsilyl), phthalidyl etc., or
- a radical which may be presented in a following form 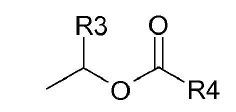
wherein R³ represents hydrogen or a lower alkyl with 1 to 4 carbon atoms, and
R⁴ represents hydrogen, alky, cycloalkyl, alkoxy, cycloalkoxy, cycloalkylalkyl, alkenyloxy, phenyl, 2-morpholinoethyl,
or a salt, ester or amide derivate of the compound of formula (I).

The compounds of formula (I) as disclosed above react as an inhibitor of beta-lactamases.

Inhibitor of beta-lactamases of formula (I) or a salt, ester or amide derivative thereof all inhibit enzymatic activity of beta-lactamases *in vitro* and enhance the potency of antibiotic agents in bacterial cell culture and are useful in particular in combination with an antibiotic for the treatment of infections in humans and animals. In contrast to other known inhibitors of beta-lactamases the inhibitor of formula (I) displays also a significant intrinsic antibiotic activity.

The invention relates to derivatives of tricyclic carbapenems of the general formula (I) in the form of pure diastereoisomers. The compounds of the formula (I) comprise at least 2 pure diastereoisomers since a new chiral centre in 4 position, which is formed in a joint point with the new ring, may be configured as (R) or as (S). The bold bond represents the position above the level of the sheet and the broken line represents the position under the level of the sheet. The mark (R) or (S) depends on the kind of ring marked C and on the substituents bound to the ring marked C and is determined according to Cahn-Ingold-Prelog rule (Calin et al., Experientia 1956, 12, 81).

The configuration in 5 position in the joint point of the four- and five- ring of the compound of the general formula I is always the same and is always under the level of the sheet and the mark (R) or (S) is determined according to the above-mentioned Cahn-Ingold-Prelog rule.

It will be appreciated that all stereoisomers including mixtures thereof arising from these additional asymmetric centres are within the scope of formula (I).

It also has been found that compounds of formula (I), when used in combination with an antibiotic, preferably in combination with beta-lactam antibiotics will result in an increased antibacterial activity (synergistic effect) against Class A, Class C and Class D producing organisms. The above mentioned combinations produced a level of inhibition of bacterial growth *in vitro* that substantially exceeded their expected additive effect.

The residue R may represent according to the present invention a hydrogen atom or a saturated alkyl chain with 1 to 20 carbon atoms and the saturated alkyl chain may be straight (such as methyl, ethyl, n-propyl, n-butyl) or branched in any position (such as isopropyl, s-butyl, isobutyl, isoamyl, tert-butyl).

Preferably, R represents an alkyl residue with 1 to 5 carbon atoms such as methyl or ethyl, in particular methyl.

The residue R1 may represent according to the present invention
- a hydrogen atom,
- a saturated alkyl chain with 1 to 20 carbon atoms and the saturated alkyl chain may be straight (such as methyl, ethyl, n-propyl, n-butyl) or branched in any position (such as isopropyl, s-butyl, isobutyl, isoamyl, tert-butyl) and each chain member may be mono or disubstituted with substituents such as halo (such as fluoromethyl, tritluoromethyl, 2-chloroethyl), hydroxy (such as hydroxymethyl, 2-hydroxyethyl), (C_{I}-C₄)-alkyloxy (such as methoxymethyl, 2-methoxyethyl), mercapto and (C_{I}-C₄)-alkylmercapto (such as mercaptomethyl, 2-methylmercaptoethyl), (C_{I}-C₄)-alkanesulfonyl (such as methanesulfonylmethyl), amino, (C_{I}-C₄)-alkylamino and di(C_{I}-C₄)-alkylamino (such as 2-aminoethyl, 2-methylaminoethyl, 2-dimethylaminoethyl), alkyleneamino (such as 2-(1-piperidinyl)ethyl, 1-pyrrolidinylmethyl), guanidino (such as guanidinomethyl), unsubstituted N¹-mono, N³-mono, N¹,N³-di and N³,N³-di-(C₁-C₄)-formamidino (such as iminomethylaminomethyl, 2-(dimethylaminomethyleneamino)ethyl), aromatic or heteroaromatic five- or six-membered ring (such as phenyl, furyl, 2-pyridyl), (C_{I}-C₄)-alkyloxycarbonyl (such as carbethoxymethyl), cyano (such as 2-cyanoethyl), oxo (such as acetyl, propionyl, 2-oxopropyl),
- an unsaturated alkyl chain with 1 to 20 carbon atoms and the unsaturated alkyl chain may be straight with double bonds or triple bonds (such as vinyl, propenyl, allyl, ethinyl, propargyl) or branched in any position with double bonds or triple bonds (such as 2-propenyl) and each chain member may be mono or disubstituted with substituents such as halo, hydroxy, (C_{I}-C₄)-alkyloxy, thio and (C_{I}-C₄)-alkylthio, (C_{I}-C₄)-alkanesulfonyl, amino, (C_{I}-C₄)-alkylamino and di-(C_{I}-C₄)-alkylamino, aromatic or heteroaromatic five- or six-membered ring (such as phenyl, furyl, 2-pyridyl), (C_{I}-C₄)-alkyloxycarbonyl, cyano, oxo,
- a saturated or partly unsaturated cycloalkyl radical with 3 to 7 members (such as radicals from cyclopropyl to cycloheptyl, cyclohex-I-enyl) and the ring may comprise one or more oxygen, sulfur or nitrogen atoms (such as 2-tetrahydrofuranyl, 1-piperidinyl, 1-pyrrolidinyl) and each ring member may be mono or disubstituted with substituents such as halo, hydroxy, (C_{I}-C₄)-alkyloxy, thio and (C_{I}-C₄)-alkylthio), (C_{I}-C₄)-alkanesulfonyl, amino, (C_{I}-C₄)-alkylamino and di-(C_{I}-C₄)-alkylamino, (C_{I}-C₄)-alkyloxycarbonyl, cyano. oxo,
- an aromatic or heteroaromatic five- or six-membered ring (such as furyl, pyranyl),
- an alkanoyl (such as formyl, acetyl, benzoyl, ethoxycarbonyl, allyloxycarbonyl, pivaloyl), an alkenoyl (such as allylcarbonyl), an aroyl (such as p-nitrobenzoyl), an alkoxycarbonyl (such as t-buthoxycarbonyl), a haloalkoxy-carbonyl (such as 2,2,2-trichloroethoxycarbonyl, or 1,1,1-trichloro-2-methyl-2propoxycarbonyl), an aralkyloxycarbonyl (such as benzyloxycarbonyl or p-nitrobenzyloxycarbonyl), an alkenyloxycarbonyl (such as allyloxycarbonyl) mono, di or tri-(C_{I}-C₄)-alkylsilyl (such as trimethylsilyl, tert-butyldimethylsilyl) group,
- a (C_{I}-C₄)-alkanesulfonyl group (such as methanesulfonyl, ethanesulfonyl), a (C_{I}-C₄)-alkenesulfonyl group (such as allylsulfonyl), arylsulfonyl group (such as p-nitrobenzylsulfonyl);

Preferably, R1 represents an alkyl residue with 1 to 5 carbon atoms such as methyl or ethyl, in particular methyl.

According to the present invention, the residue R2 may represent
- a hydrogen atom,
- an alkali metal,
- an earth alkali metal,
- the ammonium ion or a protonated form of mono, di or trisubstituted acyclic or cyclic aliphatic amine or a protonated form of some other nitrogen base,
- the quaternized ammonium ion,
- a (C_{I}-C₂₀)-alkyl (such as methylethyl, tert-butyl), (C_{I}-C₂₀)-alkenyl (such as allyl), substituted alkyl (such as (C_{I}-C₄)-alkoxyalkyl, (C_{I}-C₄)-alkylthioalkyl, phenetyl, 2,2,2-trichloroethyl, 2-oxo-5-methyl-1,3-dioxolene-4-yl)methyl, benzyl, p-methoxybenzyl, p-nitrobenzyl, o-nitrobenzyl, bis(methoxyphenyl)methyl, 3,4-dimethoxybenzyl, benzhydryl, trityl, 2-trimethylsilylethyl), substituted silyl (such as trimethylsilyl, tert-butyldimethylsilyl), phthalidyl etc., or
- a radical which may be presented in a following form 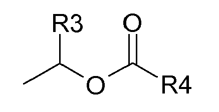 wherein R³ represents hydrogen or a lower alkyl with 1 to 4 carbon atoms, and
   R⁴ represents hydrogen, alky, cycloalkyl, alkoxy, cycloalkoxy, cycloalkylalkyl, alkenyloxy, phenyl, 2-morpholinoethyl.
If R² represents a hydrogen atom the compound of the formula I is a carboxylic acid; in C1 the case of a basic centre in the molecule, a hydrogen atom is linked to it as a proton, the carboxyl group is in the anion form as carboxylate and the compound of the formula I is in the form of a zwitter ion.

If R² represents an alkali metal the compound of the formula I is an alkali metal salt (such as lithium carboxylate, sodium carboxylate, potassium carboxylate).

If R² represents an earth alkali metal the compound of the formula I is an earth alkali metal salt wherein for one bivalent metal ion there are two carboxylate anions (such as calcium dicarboxylate).

If R² represents the ammonium ion or a protonated form of mono, di or trisubstituted acyclic or cyclic aliphatic amine or a protonated form of some other nitrogen base, the compound of the formula I is a salt of a carboxylic acid and ammonia or amine (such as trimethylamine, triethylamine, N,N'-dibenzylethylene diamine, ethanolamine, diethanolamine, triethanolamine, dicyclohexylamine, 2-piperidinyl) or amidine (such as 1,8-diazabicyclo[5.4.0]undec7-ene, 1,5-diazabicyclo[4.3.0]non-5-ene, 3,3,6,9,9-pentadimethyl-2,10-diazabicyclo[4.4.0]dec-I-ene) or guanidine (such as guanidine, cyanoguanidine) or some other nitrogen base (such as 4-dimethylaminopyridine, imidazole).

If R² represents the quaternized ammonium ion the compound of the formula I is a corresponding quaternary ammonium carboxylate (such as tetrabutylammonium carboxylate).

If R² represents a group selected from the group comprising (C_{I}-C₂₀)-alkyl (such as methylethyl, tert-butyl), (C₁-C₂₀)-alkenyl (such as allyl), substituted alkyl (such as (C_{I}-C₄)-alkoxyalkyl, (C_{I}-C₄)-alkylthioalkyl, phenetyl, 2,2,2-trichloroethyl, 2-oxo-5-methyl-1,3-dioxolene-4-yl)methyl, benzyl, p-methoxybenzyl, p-nitrobenzyl, o-nitrobenzyl, bis(methoxyphenyl)methyl, 3,4-dimethoxybenzyl, benzhydryl, trityl, 2-trimethylsilylethyl), substituted silyl (such as trimethylsilyl, tert-butyldimethylsilyl), phthalidyl etc., or a radical which may be presented in a following form 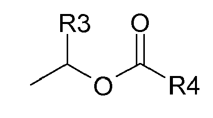 wherein R³ represents hydrogen or a lower alkyl with 1 to 4 carbon atoms, and

R⁴ represents hydrogen, alky, cycloalkyl, alkoxy, cycloalkoxy, cycloalkylalkyl, alkenyloxy, phenyl, 2-morpholinoethyl, the compounds of the formula (I) are biologically degradable esters, which are known from the group of cefalosporin antibiotics as prodrug agents (such as 1-pivaloyloxymethyl, 1-pivaloyloxyethyl, acetoxyethyl, 1-acetoxyethyl, 1-methoxy-methylethylcarbonyloxymethyl, 1-(1-methoxy-1-methylethylcarbonyloxy)ethyl, 1-benzoyloxyethyl, 1-(isopropoxycarbonyloxy)-ethyl, 1-cyclohexyloxy-carbonyloxymethyl 1-(4-ethylcyclohexyloxy-carbonyloxy)ethyl or more particularly I-, cyclohexyloxycarbonyloxyethyl esters).

Preferably, R² represents hydrogen, an alkali or earth alkali metal, in particular Na, Ca, K, or an ammonium ion or a group selected from the group comprising (C_{I}-C₂₀)-alkyl (such as methylethyl, tert-butyl), (C₁-C₂₀)-alkenyl (such as allyl), substituted alkyl (such as (C_{I}-C₄)-alkoxyalkyl, (C_{I}-C₄)-alkylthioalkyl, phenetyl, 2,2,2-trichloroethyl, 2-oxo-5-methyl-1,3-dioxolene-4-yl)methyl, benzyl, p-methoxybenzyl, p-nitrobenzyl, o-nitrobenzyl, bis(methoxyphenyl)methyl, 3,4-dimethoxybenzyl, benzhydryl, trityl, 2-trimethylsilylethyl), substituted silyl (such as trimethylsilyl, tert-butyldimethylsilyl), phthalidyl etc., or a radical which may be presented in a following form 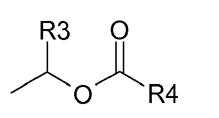 wherein R³ represents hydrogen or a lower alkyl with 1 to 4 carbon atoms, and

R⁴ represents hydrogen, alky, cycloalkyl, alkoxy, cycloalkoxy, cycloalkylalkyl, alkenyloxy, phenyl, 2-morpholinoethyl, the compounds of the formula (I) are biologically degradable esters, which are known from the group of cefalosporin antibiotics as prodrug agents (such as 1-pivaloyloxymethyl, 1-pivaloyloxyethyl, acetoxyethyl, 1-acetoxyethyl, 1-methoxy-methylethylcarbonyloxymethyl, 1-(1-methoxy-1-methylethylcarbonyloxy)ethyl, 1-benzoyloxyethyl, 1-(isopropoxycarbonyloxy)-ethyl, 1-cyclohexyloxy-carbonyloxymethyl 1-(4-ethylcyclohexyloxy-carbonyloxy)ethyl or more particularly I-, cyclohexyloxycarbonyloxyethyl esters)

According to a preferred embodiment, the compound according to formula (I) is an acid, a salt or an ester.

According to a preferred embodiment of the present invention, R and R1 each represent a methyl group. It is further preferred that R and R1 each represent a methyl group and the residue R2 represents an optionally functionalized hydrocarbon.

According to another embodiment, the present invention therefore provides a pharmaceutical composition as disclosed above, wherein in formula (I), R represents a methyl group, or R¹ represents a methyl group or R and R¹ represent a methyl group.

Furthermore, the present invention is also directed to a compound of the general formula (I) 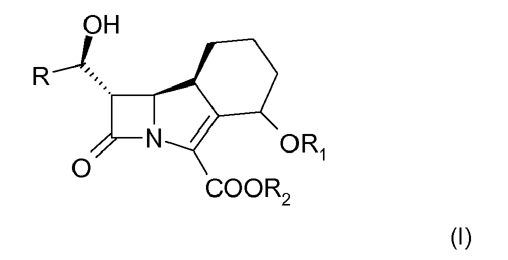 wherein
R represents a methyl group,
R1 represents a methyl group, and
R2 represents
- a (C_{I}-C₂₀)-alkyl (such as methylethyl, tert-butyl), (C₁-C₂₀)-alkenyl (such as allyl), substituted alkyl (such as (C_{I}-C₄)-alkoxyalkyl, (C_{I}-C₄)-alkylthioalkyl, phenetyl, 2,2,2-trichloroethyl, 2-oxo-5-methyl-1,3-dioxolene-4-yl)methyl, benzyl, p-methoxybenzyl, p-nitrobenzyl, o-nitrobenzyl, bis(methoxyphenyl)methyl, 3,4-dimethoxybenzyl, benzhydryl, trityl, 2-trimethylsilylethyl), substituted silyl (such as trimethylsilyl, tert-butyldimethylsilyl), phthalidyl etc., or
- a radical which may be presented in a following form 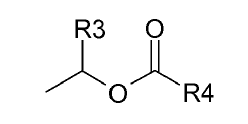 wherein R³ represents hydrogen or a lower alkyl with 1 to 4 carbon atoms, and
   R⁴ represents hydrogen, alky, cycloalkyl, alkoxy, cycloalkoxy, cycloalkylalkyl, alkenyloxy, phenyl, 2-morpholinoethyl.

These compounds can be prepared by any suitable method known to the person skilled in the art.

The present invention also relates to pharmaceutical compositions comprising at least a compound defined by formula (I) or a pharmaceutically acceptable salt, ester or amid derivative thereof and an antibiotic.

The term "antibiotic" as used herein describes a compound or composition which decreases the viability of a microorganism, or which inhibits the growth or reproduction of a microorganism. "Inhibits the growth or reproduction" means increasing the generation cycle time by at least 2-fold, preferably at least 10-fold, more preferably at least 100-fold, and most preferably indefinitely, as in total cell death. An antibiotic is further intended to include an antimicrobial, bacteriostatic, or bactericidal agent. Non-limiting examples of antibiotics useful according to the present invention include penicillins, cephalosporins, aminoglycosides, sulfonamides, macrolides, tetracyclins, lincosides, quinolones, chloramphenicol, vancomycin, metronidazole, rifampin, isoniazid, spectin-omycin, trimethoprim, sulfamethoxazole, and others.

In a preferred embodiment of the invention the antibiotic is a beta-lactam antibiotic.

The term "beta-lactam antibiotic" as used herein designates compounds with antibiotic properties containing a beta-lactam functionality.

Therefore, the present invention is also directed to a pharmaceutical composition as disclosed above, wherein the antibiotic is an beta-lactam antibiotic.

In general all beta-lactam antibiotics known by a person skilled in the art are suitable for their use within the pharmaceutical composition according to the present invention, e.g. penicillins, cephalosporins, penems, carbapenems, and monobactams.

Therefore, a preferred embodiment according to the present invention is directed towards a pharmaceutical composition as mentioned above wherein the beta-lactam antibiotic is selected from a group consisting of cephalosporins, penicillins, monobactams or carbapenems.

Examples of suitable beta-lactam antibiotics for use in the medicaments of the invention include amoxycillin, ampicillin, azlocillin, aztreonam, cefazolin, ceftazidime, cefuroxime, cefaclor, cefotaxime, ceftriaxone, ceftizaxime, cefoperazone, cefepime, cefpirome, cefmenoxime, cefoxitin, cefixime, cefpodoxime, ceftibuten, cefprozil, cephalexin, cephaloridine, ertapenem, imipenem, mecillinam, meropenem, methicillin, moxolactam, oxacillin, panipenem, penicillin G or V, piperacillin and ticarcillin.

Especially cefepime, cefpirome, ceftazidime or cefotaxime show a broad spectrum of activity against Gram-positive and Gram-negative pathogens.

Thus, the present invention also relates to a pharmaceutical composition as defined above wherein the beta-lactam antibiotic cefalosporine is selected from a group consisting of ceftazidime, cefotaxime, cefepime, cefpirome, ceftobiprole or ceftaroline.

A further embodiment of the present invention is directed towards the pharmaceutical composition as described above wherein the beta-lactam antibiotic penicilline is piperacillin.

Another preferred embodiment of the present invention is directed towards a pharmaceutical composition as mentioned above wherein the beta-lactam antibiotic monobactam is aztreonam.

Within a further preferred embodiment of the present invention the beta-lactam antibiotic carbapenem is meropenem.

The preferred beta-lactam antibiotics for combinations with the compounds of formula (I) are various cephalosporins which are divided in several generations as noted below.

First generation cephalosporins, such as cefacetrile, cefadroxil, cefalexin, cefalotin, cefamandole, cefapirin, cefazolin, ceforanide, are moderate spectrum agents, with a spectrum of activity that includes penicillinase-producing, methicillin-susceptible staphylococci and streptococci, though they are not the drugs of choice for such infections. They also have activity against some *Escherichia coli, Klebsiella pneumoniae* and *Proteus mirabilis,* but have no activity against Bacteroides fragilis, enterococci, methicilllin-resistant staphylococci, *Pseudomonas, Acinetobacter, Enterobacter,* indole-positive *Proteus* or *Serratia* ssp.

The second generation cephalosporins, such as cefaclor, cefonicid, cefradine, cefprozil, cefuroxime, cefuzonam, cefmetazole, cefotetan, cefoxitin, have a greater Gram-negative spectrum while retaining some activity against Gram-positive cocci. They are also more resistant to beta-lactamase.

Third generation cephalosporins, such as cefdinir, cefditoren, cefixime, cefoperazone, cefotaxime, cefpodoxime, ceftibuten, ceftizoxime, ceftriaxone, ceftazidime, have a broad spectrum of activity and further increased activity against Gram-negative organisms. Some members of this group, particularly those available in an oral formulation, and those with anti-pseudomonal activity, have decreased activity against Gram-positive organisms. They may be particularly useful in treating hospital-acquired infections, although increasing levels of extended-spectrum beta-lactamases are reducing the clinical utility of this class of antibiotics. Some are active also against Pseudomonas aeruginosa.

Fourth generation, such as cefepime, cefoselis, cefpirome, cephalosporins are extended-spectrum agents with similar activity against Gram-positive organisms as first-generation cephalosporins. They also have a greater resistance to beta-lactamases than the third generation cephalosporins. Many can cross the blood brain barrier and are effective in meningitis. They are also used against Pseudomonas aeruginosa.

The pharmaceutical composition of the present invention may also comprise further compounds such as conventional non-toxic pharmaceutically acceptable carrier, adjuvants or vehicles. Preferably, the compounds used in the pharmaceutical compositions of the invention are formulated in pharmaceutical compositions by combining the compounds with any conventional non-toxic pharmaceutically acceptable carrier, adjuvants or vehicles.

Thus, the present invention is also directed to a pharmaceutical composition comprising a inhibitor of beta-lactamases of formula (I) or a salt, an ester or an amide derivative thereof, an antibiotic, preferably a beta-lactam antibiotic, and a pharmaceutically acceptable carrier.

As used herein, the term "pharmaceutically acceptable" means a non-toxic material that does not interfere with the potency of the biological activity of the active ingredient(s). The term "physiologically acceptable" refers to a non-toxic material that is compatible with a biological system such as a cell, cell culture, tissue, or organism.

The term "pharmaceutically acceptable carrier" refers to a non-toxic carrier that may be administered to a patient, together with a compound of this invention in combination with antibiotics, preferably beta-lactam antibiotics, and which does not destroy the pharmacological activity thereof.

In general, all carriers known by a respective person skilled in the art are suitable for their use within the present pharmaceutical composition. Normally, the characteristics of the preferred carrier depend on the route of administration of the respective pharmaceutical composition.

Solid carriers which are usable according to the present invention are for example finely divided solids such as talc, clay, microcrystalline cellulose, silica, alumina and the like. Useful liquid carriers include water, alcohols or glycols or water- alcohol/glycol blends, in which the present compounds can be dissolved or dispersed at effective levels, optionally with the aid of non-toxic surfactants. Adjuvants such as fragrances and additional antimicrobial agents can also be added to optimize the properties for a respective use. The resultant liquid pharmaceutical compositions can be applied from an absorbent pad, used to impregnate bandages and other dressings, or sprayed onto the affected area using pump-type or aerosol sprayers.

For topical administration, it will generally be desirable to administer the present compounds to the skin as compositions or formulations, in combination with a dermatologically acceptable carrier, which may be a solid or a liquid. Topical applications may be formulated in carriers such as hydrophobic or hydrophilic bases to form ointments, creams, lotions, in aqueous, oleaginous or alcoholic liquids to form paints or in dry diluents to form powders. Thickeners such as synthetic polymers, fatty acids, fatty acid salts and esters, fatty alcohols, modified celluloses or modified mineral materials can also be employed with liquid carriers to form spreadable pastes, gels, ointments, soaps, and the like, for application directly to the skin of the user. Cream or ointment formulations which may be used for the drug are conventional formulations well known in the art.

The pharmaceutical composition of the present invention may also comprise a pharmaceutically acceptable excipient. Therefore, the present invention is also directed to a pharmaceutical composition as disclosed above, wherein the pharmaceutical composition additionally comprises a pharmaceutically acceptable excipient.

In general all excipients known by a person skilled in the art are suitable within the present invention. Examples of such excipients are calcium carbonate, kaolin, sodium hydrogen carbonate, lactose, D-mannitol, starches, crystalline cellulose, talc, granulated sugar, porous substances, etc.

The compounds of formula (I) of the invention or the salts thereof may be used as bulk itself but usually be formulated into pharmaceutical preparations together with a suitable amount of "carrier for pharmaceutical preparation" according to ordinary methods.

Thus, compositions and methods according to the invention may also contain additionally diluents, fillers, salts, buffers, stabilizers, solubilizers, and other materials well known in the art.

Further on, "carriers for pharmaceutical preparation" comprises, for example, excipients as defined above, binders, e.g., dextrin, gums, α-starch, gelatin, hydroxypropyl cellulose, hydroxypropylmethyl cellulose, pullulan, etc., thickening agents, e.g., natural gums, cellulose derivatives, acrylic acid derivatives, etc., disintegrators, e.g., carboxymethyl cellulose, croscarmellose sodium, crospovidone, low-substitution hydroxypropyl cellulose, partial α-starch, etc., solvents, e.g., water for injections, alcohol, propylene glycol, macrogol, sesame oil, corn oil, etc., dispersants, e.g., Tween 80, HCO60, polyethylene glycol, carboxymethyl cellulose, sodium alginate, etc., solubilizers, e.g., polyethylene glycol, propylene glycol, D-mannitol, benzyl benzoate, ethanol, trisaminomethane, triethanolamine, sodium carbonate, sodium citrate, etc., suspending agents, e.g., stearyl triethanolamine, sodium lauryl sulfate, benzalkonium chloride, polyvinyl alcohol, polyvinylpyrrolidone, hydroxyethyl cellulose, etc., pain-reducing agents, e.g., benzyl alcohol, etc., isotonizing agents, e.g., sodium chloride, glycerin, etc., buffers, e.g., phosphates, acetates, carbonates, citrates, etc., lubricants, e.g., magnesium stearate, calcium stearate, talc, starch, sodium benzoate, etc., colorants, e.g., tar pigments, caramel, iron sesquioxide, titanium oxide, riboflavins, etc., tasting agent, e.g., sweeteners, flavors, etc., stabilizers, e.g., sodium sulfite, ascorbic acid, etc., preservatives, e.g., parabens, sorbic acid, etc., and the like.

Pharmaceutical compositions according to the present invention may also comprise other active factors and/or agents which enhance the inhibition of beta-lactamases and/or DD-peptidases.

The respective pharmaceutical compositions are effective against bacteria which do not produce beta-lactamases, but also especially effective against bacteria which produce significant amounts of beta-lactamases. Thus, pharmaceutical compositions according to the present invention are generally useful for controlling bacterial infections levels *in vivo* and for treating diseases or reducing the advancement or severity of effects, which are mediated by bacteria.

The invention also provides methods for inhibiting bacterial growth. Methods according to the invention comprise administering a inhibitor of beta-lactamases of formula (I) in combination with antibiotics, preferably beta-lactam antibiotics to a bacterial cell culture, or to a bacterially infected cell culture, tissue, or organism.

Suitable subjects for the administration of the formulation of the present invention include mammals, primates, man, and other animals. Typically the animal subject is a mammal, generally a domesticated farm mammal, e.g. horse, pig, cow, sheep, goat etc., or a companion animal, e.g. cat, dog etc.. *In vitro* antibacterial activity is predictive of *in vivo* activity when the compositions are administered to a mammal infected with a susceptible bacterial organism.

### Route of administration

Preferred methods of administration of the pharmaceutical compositions described above include oral and parenteral, e.g., i.v. infusion, i.v. bolus and i.m. injection formulated so that a unit dosage comprises a therapeutically effective amount of each active component or some submultiples thereof.

The compounds may be employed in powder or crystalline form, in liquid solution, or in suspension. Theses compounds may be formulated by any method well known in the art and may be prepared for administration by any route, including, without limitation, parenteral, oral, sublingual, by inhalation spray, transdermal, topical, intranasal, intratracheal, intrarectal via ophthalmic solution or ointment, rectally, nasally, buccally, vaginally or via implanted reservoir. The term parenteral as used herein includes subcutaneous, intracutaneous, intravenous, intramuscular, intra-articular, intrasynovial, intrasternal, intrathecal, intralesional and intracranial injection or infusion techniques.

In case the compound of formula (I) is an acid, the pharmaceutical composition according to the present invention is preferably administered parenteral , in particular intravenous. In case the compound of formula (I) is an ester, the pharmaceutical composition according to the present invention is preferably administered orally.

Pharmaceutical compositions for injection, a preferred route of delivery according to the present invention, may be prepared in unit dosage form in ampules, or in multidose containers. The composition will generally be sterile and pyrogen-free, when intended for delivery by injection into the subject. The injectable compositions may take such forms as suspensions, solutions, or emulsions in oily or aqueous vehicles, and may contain various formulating agents. Alternatively, the active ingredient may be in powder (lyophilized or non-lyophilized) form for reconstitution at the time of delivery with a suitable vehicle, such as sterile water.

Carriers suitable for an injectable pharmaceutical composition according to the present invention are typically comprised sterile water, saline or another injectable liquid, e.g., peanut oil for intramuscular injections. Also, various buffering agents, preservatives and the like can be included. The pharmaceutical composition according to the present invention may also be administered parenterally in a sterile medium. Depending on the vehicle and concentration used, the drug can either be suspended or dissolved in the vehicle. Advantageously, adjuvants such as local anaesthetic, preservative and buffering agents can be dissolved in the vehicle. The proper fluidity can be maintained, for example, by the formation of liposomes, by the maintenance of the required particle size in the case of dispersions or by the use of surfactants. The prevention of the action of microorganisms can be brought about by various antibacterial and antifungal agents, for example, parabens, chlorobutanol, phenol, sorbic acid, thimerosal, and the like. It is also preferred to include isotonic agents, for example, sugars, buffers or sodium chloride. Prolonged absorption of the injectable compositions can be brought about by the use in the compositions of agents delaying absorption, for example, aluminium monostearate and gelatine. Intra-venous infusion is another possible route of administration for the compounds used according to the present invention.

Orally administrable pharmaceutical compositions according to the present invention may be in the form of tablets, capsules, powders, granules, lozenges, liquid or gel preparations, such as oral, topical, or sterile parenteral solutions or suspensions. The oral compositions may utilize carriers such as conventional formulating agents, and may include sustained release properties as well as rapid delivery forms. Such compositions and preparations should contain at least 0.1% of active compounds. The percentage of the compositions and preparations may, of course, be varied and may conveniently be between about 2 to about 60% of the weight of a given unit dosage form. The amount of active compound in such therapeutically useful compositions is such that an effective dosage level will be obtained.

Tablets and capsules for oral administration may be in unit dose presentation form, and may also contain conventional excipients such as binding agents, for example syrup, acacia, gelatine, sorbitol, tragacanth, or polyvinyl-pyrrolidone; fillers for example lactose, sugar, maize-starch, calcium phosphate, sorbitol or glycine; tabletting lubricant, for example magnesium stearate, talc, polyethylene glycol or silica; disintegrates for example potato starch, or acceptable wetting agents such as sodium lauryl sulphate. The tablets may be coated according to methods well known to a person skilled in the art. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatine hydrogenated edible fats; emulsifying agents, for example lecithin, sorbitan monooleate, or acacia; non-aqueous vehicles which may include edible oils, for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

Pharmaceutical compositions according to the present invention may also be prepared in suitable forms for absorption through the mucous membranes of the nose and throat or bronchial tissues and may conveniently take the form of powder or liquid sprays or inhalants, lozenges, throat paints, etc. For medication of the eyes or ears, the preparations may be presented as individual capsules, in liquid or semi-solid form, or may be used as drops, etc.

For veterinary medicine, the composition may, for example, be formulated as an intramammary preparation in either long acting or quick-release bases.

### Use of the compound of formula (I) as a broad spectrum inhibitor of beta-lactamases

The invention also provides novel inhibitor of beta-lactamases of formula (I) described above as well as a pharmaceutical composition comprising an antibiotic and a compound of the general formula (I). Certain embodiments of these inhibitors also bind bacterial DD-peptidases (PBP-penicillin binding proteins), and thus act both as inhibitor of beta-lactamases and as antibiotic agents.

Therefore inhibitor of beta-lactamases of formula (I) can extend action of the beta-lactam antibiotic in the combination to strains producing inhibitor-sensitive enzymes and additionaly improve its antibacterial spectrum. Spectrum gain due to antibiotic activity of the inhibitor of beta-lactamases of formula (I) is deemed superior to commercially available inhibitors of beta-lactamases, such as clavulanic acid, sulbactam and tazobactam which have no antibiotic activity per se.

Compounds of formula (I) are especially suitable as inhibitors of beta-lactamases for therapeutic applications. They are also useful as pharmacological tools for *in vitro* or *in vivo* studies to investigate the mechanisms of antibiotic resistance, to help identify other therapeutic antibiotic agents or inhibitors of beta-lactamases, to identify which beta-lactamases are being expressed by a given microorganism, or to selectively inhibit one or more beta-lactamases in a microorganism.

Thus, the present invention also relates to the use of a therapeutically effective amount of inhibitor of beta-lactamases of formula (I) as defined above or a salt, ester or amide derivates thereof as a broad spectrum inhibitor of beta-lactamases.

According to a preferred embodiment the inhibitor of beta-lactamases is a broad spectrum inhibitor of class A, C and D beta-lactamases. It effectively inhibits most of the clinically relevant and prevalent TEM- and SHV-type enzymes (class A), AmpC (class C) and OXA-type enzymes (class D).

Thus, the present invention is also directed to the use of a therapeutically effective amount of one or more compounds of formula (I) as defined above or a salt, ester or amide derivative thereof as a broad-spectrum beta-lactamase inhibitor, in particular wherein the beta-lactamase inhibitor is a beta-lactamase inhibitor of class A, C and D.

The pharmaceutical composition according to the present invention comprising a broad spectrum inhibitor of beta-lactamases in combination with an antibiotic, in particular a selected beta-lactam antibiotic is clearly superior to current therapeutic options.

Additionally, the present invention according to another embodiment also provides the use of a therapeutically effective amount of one or more compounds of formula (I) as defined above or a salt, ester or amide derivative thereof as an antibiotic.

### Inhibition of Bacterial Growth

In a further aspect, the present invention provides methods for inhibiting bacterial growth, such methods comprising administering a pharmaceutical composition according to the present invention comprising an inhibitor of beta-lactamases of formula (I) in combination with antibiotics, preferably a beta-lactam antibiotics as defined above to a bacterial cell culture, or to a bacterially infected cell culture, tissue, or organism.

It is known that the response to a given combination may be strain specific and is not solely related to the level of sensitivity/resistance to the specific members of the combination. Thus, the combinations of the present invention are intended to be useful on all bacterial strains including those not mentioned herein.

Preferably, the bacteria to be inhibited by administration of the pharmaceutical composition according to the present invention are bacteria that are resistant to beta-lactam antibiotics. More preferably, the bacteria to be inhibited are beta-lactamase positive strains that are highly resistant to beta-lactam antibiotics. The terms "resistant" and "highly resistant" are well-known by those of ordinary skill in the art.

Polymicrobial infections often include pathogens that produce beta-lactamase enzymes. These enzymes commonly cause resistance to penicillins and cephalosporins. Without treatment these microbes would multiply and thrive unimpeded, with serious or critical consequences to the patient.

Thus the present invention also relates to methods for overcoming bacterial antibiotic resistance.

### Method of treatment

The pharmaceutical composition according to the present invention are useful for inhibiting bacterial growth in a variety of contexts. In a preferred embodiment of the present invention, the pharmaceutical composition according to the present invention is administered to an experimental cell culture *in vitro* to prevent the growth of beta-lactam resistant bacteria. According to another preferred embodiments the pharmaceutical composition according to the present invention is administered to an animal, including a human, to prevent the growth of beta-lactam resistant bacteria *in vivo.* The method according to this embodiment comprises administering a therapeutically effective amount of a pharmaceutical composition according to the present invention for a therapeutically effective period of time to an animal, including a human.

Thus, the present invention is also directed towards a method of inhibiting beta-lactamase comprising contacting the beta-lactamase with an effective amount of inhibitor of beta-lactamases of formula (I) defined above or a salt, ester or amide derivative thereof.

According to a further embodiment the present invention provides a method of treatment of a bacterial infection in a human or animal subject wherein the method comprising administering to the subject in need thereof a therapeutically effective amount of inhibitor of beta-lactamases of formula (I) defined above or a salt, ester or amide derivative thereof, and an antibiotic, preferably a beta-lactam antibiotic.

Thus, the present invention is also directed towards the use of a pharmaceutical composition as described above for the treatment of an infection in humans or animals caused by a bacterium.

The present invention is also directed to the use of a pharmaceutical composition as disclosed above for the treatment of an infection in humans or animals caused by bacteria.

Additionally, the present invention is directed to a method of treating an infection in humans or animals caused by bacteria comprising administering to a patient in need of such treating a therapeutically effective amount of the composition according to the present invention and at least one pharmaceutically acceptable excipient.

Finally, the present invention is also directed to the use of a therapeutically effective amount of the composition according to the present invention and at least one pharmaceutically acceptable excipient for the preparation of a medicament for treating an infection caused by bacteria, preferably wherein said medicament is to be administered to a patient in need thereof.

Safe and effective dosages for different classes of patients and for different disease states will be determined by clinical trial as is required in the art. The specific dosage and treatment regimens for any particular patient will depend upon a variety of factors, including the activity of the specific compound employed, the age, body weight, general health status, sex, diet, time of administration, route and frequency of administration, rate of excretion, drug combination, the sensitivity of the pathogen to the particular compound selected, the virulence of the infection, the severity and course of the disease, and the patient's disposition to the disease. Such matters, however, are left to the routine discretion of the physician according to principles of treatment well known in the antibacterial arts.

The terms "therapeutically effective amount" and "therapeutically effective period of time" are used to denote known treatments at dosages and for periods of time effective to show a meaningful patient benefit, i.e., healing of conditions associated with bacterial infection, and/or bacterial drug resistance. Preferably, such administration should be parenteral, oral, sublingual, transdermal, topical, intranasal, intratracheal, or intrarectal. When administered systemically, the therapeutic composition is preferably administered at a sufficient dosage to attain a blood level of inhibitor of at least about 0.1 mg/mL, more preferably about 1 mg/mL, and still more preferably about 10 mg/mL. For localized administration, much lower concentrations than this may be effective, and much higher concentrations may be tolerated.

In case of co-administration of inhibitor of beta-lactamases of formula (I) as defined above or a salt, ester or amide derivative thereof with an antibiotic, most preferably a beta-lactam antibiotic, the ratio of the amount of the compound to the amount of the antibiotic, most preferably a beta-lactam antibiotic may vary in a wide range.

The ratio of beta-lactam antibiotic to inhibitor of beta-lactamases of formula (I) may vary from 1:1 to 100:1. Preferably the ratio of the beta-lactam antibiotic to inhibitor of beta-lactamases is less than 10:1, for example 4:1 or 2:1.

The pharmaceutical compositions according to the present invention for human delivery per unit dosage, whether liquid or solid, comprise from about 0.01% to as high as about 99% of inhibitor of beta-lactamases of formula (I) or derivative thereof, such as a salt, ester or amide. The preferred range being from about 10 to about 60% and from about 1% to about 99.99% of one or more of other antibiotics such as those discussed herein, preferably from about 40% to about 90%.

The pharmaceutical composition will generally contain from about 1 mg to about 2.0 g of the inhibitor of beta-lactamases of formula (I) or derivative thereof, such as a salt, ester or amide. However, in general, it is preferable to employ dosage amounts in the range of from about 1 mg to 1000 mg and from about 50 mg to about 5 g of the other antibiotics discussed herein; preferably from about 250 mg to about 2000 mg.

In parenteral administration, the unit dosage will typically include the pure inhibitor of beta-lactamases of formula (I) in sterile water solution or in the form of a soluble powder intended for solution, which can be adjusted to neutral pH and isotonic. For children, a dose of about 5-25 mg/kg of body weight given 2, 3, or 4 times per day is preferred; a dose of 10 mg/kg is typically recommended.

According to a preferred embodiments of the method according to the present invention, a inhibitor of beta-lactamases of formula (I) according to the invention is co-administered with an antibiotic, preferably with a beta-lactam antibiotic.

For purposes of this invention, the term "co-administered" is used to denote simultaneous or sequential administration. Preferably, such co-administration produces a synergistic effect. As employed herein, the terms "synergy" and "synergistic effect" indicate that the effect produced when two or more drugs are co-administered is greater than would be predicted based on the effect produced when the compounds are administered individually. [Chou and Talalay, Adv. Enzyme Regul. 1984, 22, 27-55; J Lehar et al, Mol. Systems Biol. 2007, 3, 80, 1-14; Yeh et al, Nature Gen. 2006, 38, 4, 489-494].

In general, a synergistic effect is most clearly demonstrated at sub-optimal concentrations of the compounds (i.e., sub-therapeutic dosages). A lower dosage minimizes the potential of side effects, thereby providing an increased margin of safety. Synergy can be in terms of lower cytotoxicity, increased antimicrobial effect, or some other beneficial effect of the combination compared with the individual components.

The inhibitor of beta-lactamases used according to the present invention act to prevent degradation of beta-lactam antibiotics, thereby enhancing their efficacy and producing a synergistic effect. Thus, according to a preferred embodiment of the present invention the co-administered antibiotic is a beta-lactam antibiotic.

According to a preferred embodiment of the present invention inhibitor of beta-lactamases of formula (I) as defined above are co-administered with an antibiotic selected from the group consisting of cephalosporin, penicillin, monobactam or carbapenem.

In a further preferred embodiment of the present invention the compounds of the present pharmaceutical composition are co-administered with cephalosporin, such as cefepime, cefpirome, ceftazidime, cefotaxime, ceftriaxone, cefpirome, cefoperazone, ceftaroline or ceftobiprole intravenously.

In a further preferred embodiment of the present invention inhibitor of β-lactamases of Formula (I) in form of prodrug ester as defined above are co-administered per os with cephalosporin, such as cefaclor, cefadroxil, cefalexine, cefprozile, cefpodoxime, cefuroxime axetil, cefpodoxime proxetil ceftaroline in form of N-phosphono prodrug or ceftobiprole medocaril.

The compounds of the pharmaceutical composition of the present invention may be provided prior to, simultaneously with, or subsequent to a beta-lactam antibiotic ("co-administration"). The two active components may be administered separately by different routes, if desired.

The terms "combination," "combined" and similar expressions, when used in reference to the administration of two or more compounds, mean that the compounds are administered to a subject concurrently. Concurrent administration includes administration at the same time, in the same formulation or separately, and sequential administration in any order or at different points in time so as to provide the desired therapeutic effect.

In a preferred embodiment of the present invention the two active agents will be administered by the same route and preferably in a single composition, so as to ensure that they are given simultaneously to the subject.

Although illustrative embodiments of the invention have been described in detail, it is to be understood that the present invention is not limited to those precise embodiments, and that various changes and modifications can be effected therein by one skilled in the art without departing from the scope and spirit of the invention as defined by the appended claims.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

The invention is further described in connection with the following non-limiting examples.

### EXAMPLES

### EXAMPLE 1

### Materials and Methods

### IC₅₀ Determination for the Inhibitors of beta-Lactamases of formula (I):

The IC₅₀ value represents the concentration of inhibitor required to effect a 50% loss of activity of free enzyme. A standard test for the production of beta-lactamase involves use of the chromogenic cephalosporin, nitrocefin. This compound exhibits a rapid distinctive colour change from yellow (maximum OD at pH 7.0 at lambda 390 nm) to red (maximum OD at pH 7.0, at lambda 486 nm), as the amide bond in the beta-lactam ring is hydrolysed by a beta-lactamase.

Homogeneously purified class A beta-lactamases TEM-1 and SHV-1 from *E. coli* and class C enzyme P99 from *Enterobacter cloaca* were employed in the assay.

All the enzymes and compounds were dissolved in 50 mM phosphate buffer pH 7.0 and all further dilutions were done with the same buffer solution. Enzyme and compound dilutions were pre-incubated for 30 min at 37°C and in a final volume of 500 µl. Than 10 µl of 5 mM nitrocefin (reporter substrate) was added to the solution and the absorbance at 482 nm was measured during 2 to 5 minutes. The initial rate was calculated for all the different solutions. The IC₅₀ values were determined as the inhibitor concentration that gave an initial hydrolysis rate of nitrocefin equal to 50 % of the hydrolysis rate of nitrocefin in absence of inhibitor.

Representative compounds of formula (I) were evaluated as inhibitors of beta-lactamases of TEM-1 and SHV-1 (class A, penicillinase) from E. *coli* and P-99 (class C, cephalosporinase) from *Enterobacter cloacae,* by relative IC₅₀ analysis using a procedure similar to that described above. The data is presented in Table 1 below.

**Table I: In vitro IC₅₀ activities of inhibitors of beta-lactamases LK-176, LK-177 and LK-179 against class A (TEM-1 and SHV-1) and class C (P99) beta-lactamases.**

| | | IC₅₀ [µM] | | |
|---|---|---|---|---|
| Compound | R1 | TEM-1 | SHV-1 | P99 (AmpC) |
| LK-176 | - Me | 0.015 | 0.2 | 0.00015 |
| LK-177 | -(CH₂)₂F | 7.4 | 223 | 0.149 |
| LK-179 | - Et | 785 | 2830 | 0.251 |

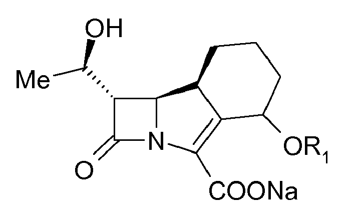

(4*S*,8*S*,9*R*)-4-methoxy-10-(1-Hydroxyethyl)-11-oxo-1-azathcyclo-[7.2.0.0 (3,8)]undec-2-ene-2-carboxylic acid, (thereafter referred to as "LK-176") a pharmaceutically acceptable salt or ester thereof was selected from a series of inhibitors of beta-lactamases of formula (I) as a promising inhibitor of beta-lactamases to be co-administered with a selected beta-lactam antibiotic.

### EXAMPLE 2

Synergistic Effect of LK-176 when Tested in Combinations with Ceftazidime, Cefotaxime, Cefepime, Cefuroxime, Ceftriaxone and Piperacillin Against Class A and Class C beta-Lactamase Positive Bacterial Strains

Representative inhibitor of beta-lactamases of formula (I) in combinations with ceftazidime, cefotaxime, cefepime, cefuroxime, ceftriaxone and piperacillin was tested in microdilution susceptibility asssay (Table 2) and compared with the commercially available combination product Tazocin^{®} (tazobactam/piperacillin).

### Antibiotics

Stock solutions of the test compounds and Tazocin^{®} were prepared in distilled water according to the CLSI guidelines [Methods for dilution antimicrobial tests for bacteria that grow aerobically. NCCLS document M7-A5; 2000; vol. 19. Clinical and Laboratory Standards Institute, Villanova, Pa.].

Combinations of different beta-lactam antibiotics (ceftazidime, cefotaxime, cefepime, cefuroxime, ceftriaxone and piperacillin) and inhibitor of beta-lactamases of formula (I) with constant concentration ratio (2:1) and (10:1) were tested and compared to beta-lactam antibiotics alone and Tazocin^{®}.

### Bacterial strains

All tested strains and clinical isolates were either purchased from the American Type Culture Collection (ATCC), or from the in-house company culture collection. The tested strains were purely used for the purposes of illustrative example, they are by no means essential for performing the invention.

Representative inhibitor of beta-lactamases of formula (I) was evaluated against the bacterial strains producing serine-based class A beta-lactamases including CTX-M, TEM-type, SHV-type extended spectrum beta-lactamases (ESBL) and class C beta-lactamases (AmpC) as noted in Table 2.

### Cultivation and Maintenance of Test Organisms

The strains were processed according to procedures recommended by ATCC, or procedures that are routinely used. Frozen bacterial stocks were thawed to room temperature, and a few drops were placed on an appropriate blood agar plate. The cultures were subcultured on a fresh Mueller-Hinton agar plate (MHA) the following day, and the subcultures were again incubated overnight.

### Inoculum

Bacterial suspensions with a turbidity equivalent to that of a 0.5 McFarland standard were prepared by suspending a tiny portion of one colony from blood agar plates in 2 mL of sterile saline. Suspensions were further diluted with cation adjusted Mueller Hinton Broth (CAMHB) to obtain a final inoculum of 5 x 10⁵ CFU/ mL.

### Assay Procedure

The *in vitro* activities of the antibiotics were determined by the broth microdilution method as recommended by CLSI guidelines.

MIC experiments were performed in duplicate in 96-well microtiter plates using CAMHB. Serial twofold dilutions of each antibiotic either alone or in combination with constant concentration ratio of LK-176 (2:1) and (10:1) were prepared in CAMHB. The bacterial suspension with final inoculum 10⁵ CFU/mL was transferred to the test medium containing the antibacterial substances. In each well of microtiter plate 50 µL of bacterial inoculum and 50 µL of antibiotic dilutions were combined. Each plate included 4 wells with no bacterial inoculum (negative control) and 4 wells with no test compound and no antibiotic (positive control). The plates were incubated at 35°C for 24 h. Purity check and colony counts on each inoculum suspension was performed to ensure that the final inoculum concentration routinely obtained closely approximates 5 x 10⁵ CFU/mL.

In addition the assay is routinely monitored by testing standard antibiotics and ensuring that MIC values are within the recommended ranges for the respective control strains.

The minimal inhibitory concentration (MIC) for all isolates was defined as the lowest concentration of antimicrobial agent that completely inhibits the growth of the organism as detected by the unaided eye.

This test permits comparisons to be made between bacterial growth in the presence of antibiotic alone and bacterial growth in the presence of both an antibiotic and inhibitor of beta-lactamases of formula (I). Representative results are presented in Table 2.

**Table 2. MIC values of LK-176, ceftazidime, cefotaxime, cefepime, cefuroxime, ceftriaxone, piperacillin and respective combinations ceftazidime/LK-176, cefotaxime/LK-176, cefepime/LK-176 cefuroxime/LK-176, ceftriaxone/LK-176, and piperacillin/LK-176, in concentration ratios 2:1 and 10:1 are compared to Tazocin^{®}.**

| **Compound** | **S1** | **S2** | **S3** | **S4** | **S5** | **S7** | **S8** | **S9** | **S10** |
|---|---|---|---|---|---|---|---|---|---|
| Tazocin® | 64 | 128 | 128 | 16 | 4 | 16 | 16 | 32 | 32 |
| | 32 | 128 | 128 | 16 | 4 | 8 | 16 | 32 | 32 |
| LK-176 | 32 | 64 | 64 | 32 | 32 | 16 | 128 | 64 | 32 |
| | 32 | 8 | 64 | 32 | 32 | 16 | 128 | 32 | 32 |
| Ceftazidime (TAZ) | >128 | >128 | 128 | 16 | 16 | 4 | 32 | 128 | 32 |
| | >128 | >128 | 128 | 16 | 16 | 4 | 32 | 64 | 32 |
| TAZ/LK-176=2/1 | 8 | 8 | 8 | 4 | 8 | 4 | 4 | 4 | 8 |
| | 8 | 8 | 8 | 2 | 4 | 2 | 4 | 4 | 4 |
| TAZ/LK-176=10/1 | 16 | 16 | 16 | 4 | 8 | 4 | 8 | 8 | 8 |
| | 16 | 8 | 16 | 4 | 8 | 4 | 8 | 4 | 8 |
| Cefotaxime (OTA) | 128 | 128 | 64 | 8 | 16 | 32 | 4 | 32 | 16 |
| | 128 | 128 | 64 | 8 | 16 | 32 | 2 | 32 | 16 |
| OTA/LK-176=2/1 | 8 | 8 | 8 | <1 | <1 | 8 | <1 | 2 | 4 |
| | 4 | 8 | 8 | <1 | <1 | 4 | <1 | 2 | 4 |
| OTA/LK-176=10/1 | 16 | 16 | 16 | <1 | <1 | 8 | <1 | 4 | 8 |
| | 8 | 16 | 16 | <1 | <1 | 4 | <1 | 2 | 8 |
| Cefepime (EPI) | 8 | 8 | 8 | 4 | 32 | 16 | 2 | 8 | 4 |
| | 8 | 8 | 8 | 4 | 16 | 16 | 2 | 8 | 4 |
| EPI/LK-176=2/1 | 2 | 4 | <1 | <1 | 16 | 8 | <1 | 2 | <1 |
| | 2 | 4 | <1 | <1 | 8 | 4 | <1 | 2 | <1 |
| EPI/LK-176=10/1 | 2 | 4 | <1 | <1 | 8 | 4 | <1 | 4 | <1 |
| | 2 | 4 | <1 | <1 | 4 | 4 | <1 | 4 | <1 |
| | | | | | | | | | |

| **Compound** | **S1** | **S2** | **S3** | **S4** | **S5** | **S6** | **S7** | **S8** | **S9** |
|---|---|---|---|---|---|---|---|---|---|
| Tazocin® | 16 | 16 | 16 | 16 | 16 | 16 | 8 | 8 | 32 |
| | 16 | 16 | 16 | 16 | 8 | 8 | 8 | 4 | 32 |
| LK-176 | 32 | 64 | 64 | 16 | 64 | 16 | >128 | 64 | 32 |
| | 16 | 64 | 32 | 16 | 64 | 16 | 128 | 64 | 32 |
| Cefuroxime (URO) | >128 | >128 | >128 | 128 | 64 | >128 | 64 | 128 | 128 |
| | >128 | >128 | >128 | 128 | 32 | >128 | 32 | 128 | 128 |
| URO/LK-176=2/1 | 8 | 8 | 8 | 4 | 2 | <1 | 8 | 2 | 4 |
| | 4 | 8 | 8 | 4 | 2 | 2 | 8 | 2 | 4 |
| URO/LK-176=10/1 | 16 | 32 | 32 | 8 | 4 | 4 | 16 | 4 | 16 |
| | 16 | 32 | 32 | 8 | 2 | 4 | 16 | 4 | 16 |
| Piperacillin (PIP) | >128 | >128 | >128 | 32 | 16 | >128 | >128 | >128 | 128 |
| | >128 | >128 | >128 | 32 | 16 | >128 | >128 | >128 | 128 |
| PIP/LK-176=2/1 | 8 | 8 | 8 | 2 | 2 | 2 | 4 | 4 | 4 |
| | 4 | 4 | 4 | 2 | 2 | 2 | 4 | 2 | 4 |
| PIP/LK-176=10/1 | 16 | 16 | 32 | 4 | 4 | 4 | 16 | 8 | 16 |
| | 16 | 16 | 32 | 4 | 4 | 4 | 8 | 8 | 16 |
| Ceftriaxone (TRI) | 128 | 64 | 32 | 8 | >128 | >128 | 8 | 32 | 32 |
| | 128 | 64 | 32 | 8 | >128 | 128 | 8 | 32 | 16 |
| TRI/LK-176=2/1 | 4 | 4 | 4 | <1 | 2 | 2 | <1 | 2 | 4 |
| | 4 | 4 | 4 | <1 | 2 | 2 | <1 | 2 | 4 |
| TRI/LK-176=10/1 | 16 | 16 | 8 | 2 | 4 | 4 | 2 | 4 | 8 |
| | 8 | 8 | 8 | 2 | 4 | 4 | 2 | 4 | 8 |

**Table 3. List of bacterial strains used in microdilution susceptibility assay.**

| ID | Bacterial strain | No. | beta-lactamase |
|---|---|---|---|
| S1 | *E. cloacae* | B274 | Am pC |
| S2 | *E. cloacae* | B275 | Am pC |
| S3 | *E. cloacae* | 4013 | *not defined* |
| S4 | *E. caerogenes* | ATCC 29751 | class II |
| S5 | *E. faecalis* | ATCC 29212 | *not defined* |
| S6 | *K. pneumoniae* | DSA 1461 | *not defined* |
| S7 | *K. pneumoniae* | ATCC 700603 | SHV-18 |
| S8 | *C. freundii* | B271 | SHV-5 |
| S9 | *C. freundii* | B318 | CTX-M |

It can be shown by established test models and in particular those models described herein that the combination of the invention results in synergistic activity compared to the effects observed with the single combination partners.

All strains S1-S9 used were deemed as resistant to LK-176 alone with MIC values above 16 mg/L. In general cefepime and ceftriaxone were more active against tested strains as other cephalosporins and piperacillin.

As can be seen from the Table 2, the synergistic combinations of active compounds according to the present invention are markedly superior to conventionally used Tazocin^{®} and beta-lactam antibiotics alone. In combinations of LK-176 with constant concentration ratio (2:1) and (10:1) MICs were evidently reduced. Overall LK-176 consistently expressed potentiated spectrum of activity in combination with all beta-lactam cephalosporins against A beta-lactamases including CTX-M, TEM-type, SHV-type extended spectrum beta-lactamases (ESBL) and class C beta-lactamases (AmpC) producing strains. The effect was more profound when higher concentration of LK-176 (2:1) was used.

The antmicrobial effectiveness of the particular new synergistic combinations of active compounds of the present invention are substantially (and surprisingly) higher than the sum of the separate effects of the individual active compounds. In all combinations of cephalosporins with LK-176 there was significant lowering of MICs consistently observed. Overall, the lowest MICs were observed in combination of cefepime and ceftriaxone with LK-176 (2: 1).

### EXAMPLE 3

Synergistic Effect of LK-176 when Tested in Combinations with Ceftazidime, Cefotaxime and Cefepime Against Class A and Class C beta-Lactamase Positive Bacterial Strains in Broth Microdilution Assay

Representative inhibitor of beta-lactamases of formula (I) in combinations with ceftazidime and cefotaxime was tested in broth microdilution assay (Table 4 and 5), where the dynamics of bacterial killing against *Citrobacter freundii, Enterobacter cloacae,* and *Klebsiella pneumoniae* was assessed.

Combinations can be tested by the factorial design (also 'checkerboard' or 'dose matrix') where combinations are tested in all possible permutations of serially diluted single agent doses. Appropriate concentrations of both agents were diluted with concentrations ranging from 256 to 4 µg/ml for ceftazidime and cefotaxime (two-fold dilution) and from 16 to 0.0625 µg/ml for LK-176 (four-fold dilution). Log-phase bacteria were adjusted to 5x10⁵ CFU per ml (inoculum), and broth microdilution assays were performed in 96-well plates in a checkerboard fashion. The plates were incubated aerobically for 24 h at 37°C.

To evaluate interactions between agents, we calculated the fractional inhibitory concentrations (FICs). FIC index was calculated by the following formula:
FIC_{A}=(MIC_{A} in combination)/(MIC_{A} alone),
FIC_{B}=(MIC_{B} in combination)/(MIC_{B} alone), and
FIC index = FIC_{A}+FIC_{B}, where FIC_{A} (FIC_{B}) and
MIC_{A} (MIC_{B}) are the FIC and MIC for antibiotic A (B), respectively.
FIC indices were used to characterize antibiotic interactions:
Synergy if FIC index ≤0.5
Additivity if 0.5< FIC index <1
Indifference if 1< FIC index ≤4 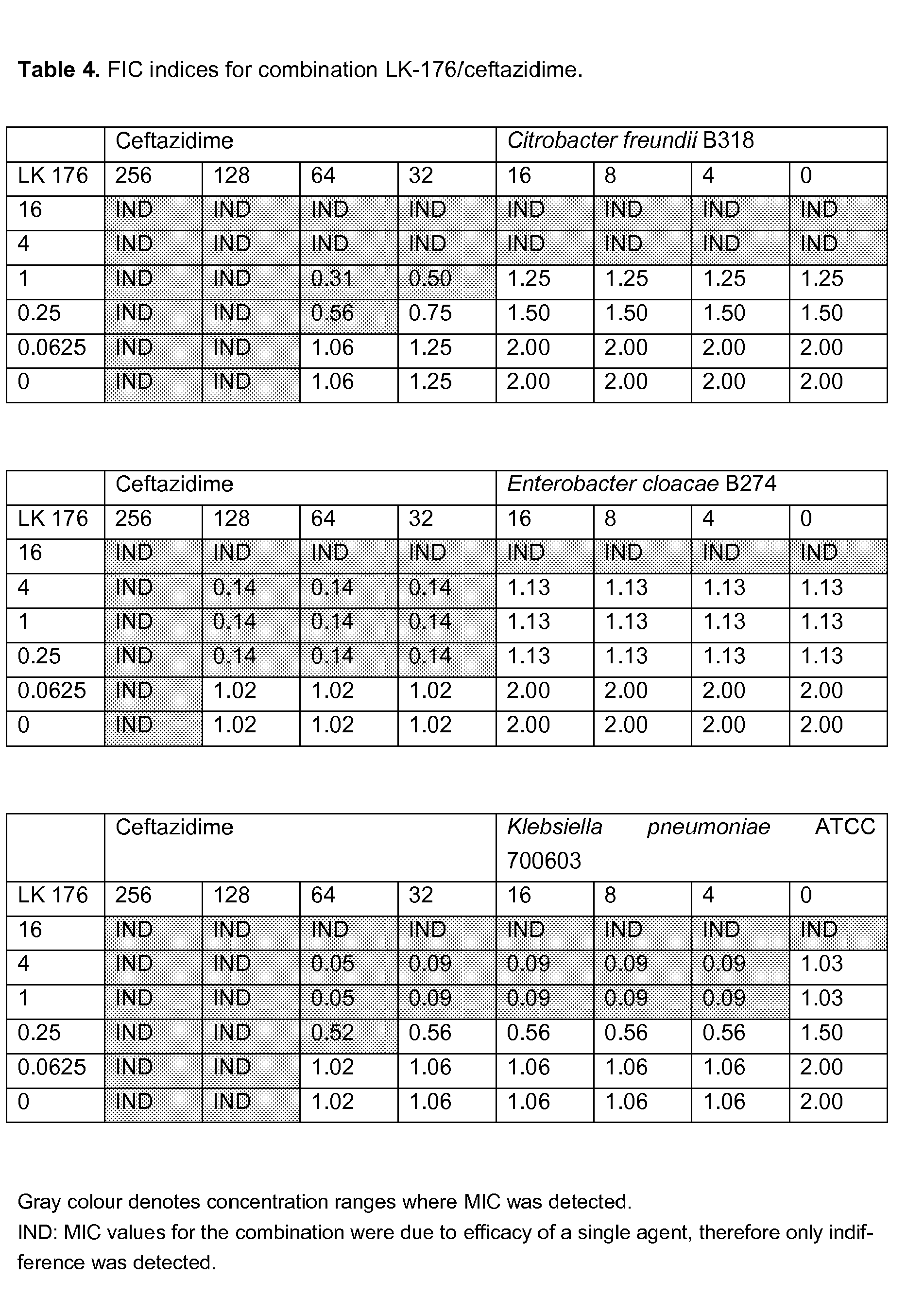 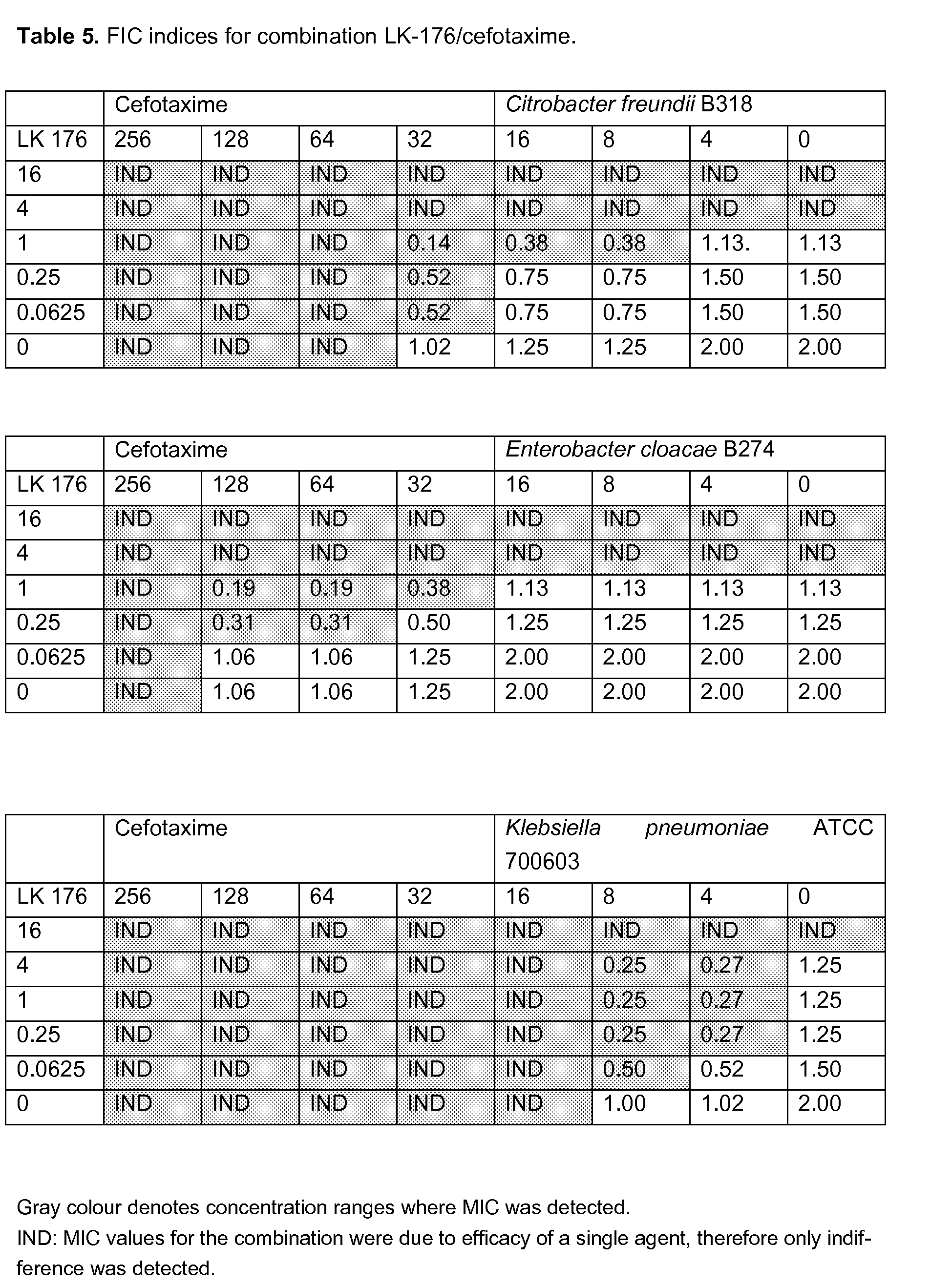 .

Two drugs are considered additive if the relative phenotypic effect of each of the drugs does not depend on the presence of the other drug. Combination responses to varying concentrations of compounds provide a more detailed look at synergistic perturbations. A synergistic effect in terms of reducing MICs was evidently observed in both combinations LK-176/cefotaxime and LK-176/ceftazidime against tested clinical isolates.

Synergy and additivity is displayed in the grey area within smaller box according to the above mentioned criteria (Table 4 and 5).

The drug combinations produced a level of inhibition that substantially exceeded their expected additive effect. These findings provide further evidence for synergistic activity consistent with that observed in susceptibility screening discussed above.

These data clearly show the synergized action of the combinations in terms of reduced MICs, even at very low dosage rates. Thus the diminished activity of partner beta-lactam antibiotic against the resistant strains was effectively restored.

Based on *in vitro* efficacy LK-176 could be administered in single doses of 0.25-4 g/day, i.v. or repeated doses of 1.5-3 g/day, i.v., and orally in single doses of 0.25-2 g/day, i.v. or repeated doses of 0.25-1 g/day, i.v.

### EXAMPLE 4

The following procedure for the preparation of inhibitor of beta-lactamases of formula (I) in preferred ester prodrug form was used.

4-(2-Chloroethyl)morpholine (CEM) was added into a suspension of LK176 in DMF and the mixture heated for 30 min at 100°C under MW irradiation. The reaction mixture was evaporated and the residue purified with extraction (dichloromethane/water) followed by dry flash chromatography (hexane/ethylacetate=1/2) to give LK-176E1 (pale yellow oil) in 46% yield (Scheme 1).

The chemical name of LK176E1 is morpholinoethyl (8R,9R)-10-(S)-[1-(R)-hydroxyethyl]-4-(R)-methoxy-11-oxo-azatricyclo-[7.2.0.0^{3,8}]undec-2-en-carboxylate.

¹H NMR (300 MHz, CDCl₃) δ 2.20-1.20 (9H, m, H-5, H-6, H-7, CH(OH)CH₃), 2.45-2.55 (4H, m, 2 × NCH₂), 2.68 (2H, t, J = 6.0 Hz, COOCH₂CH₂), 3.20-3.30 (5H, m, OCH₃, H-8, H-10), 3.65-3.75 (4H, m, 2 × OCH₂), 4.19 (1 H, m, H-9), 4.23 (1 H, m, CH(OH)), 4.25-4.45 (2H, m, COOCH₂CH₂), 4.98 (1 H, t, J = 3.0 Hz, 4-H); MS m/z 395 (M+H)⁺.

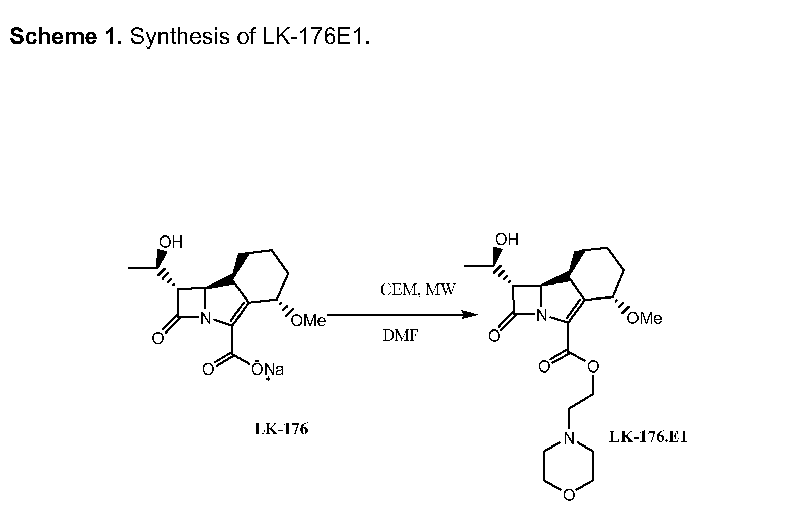

## Claims

1. A pharmaceutical composition with broad-spectrum of activity against class A, class C and D enzymes comprising an antibiotic and a pharmaceutically effective amount of a compound of formula (I) 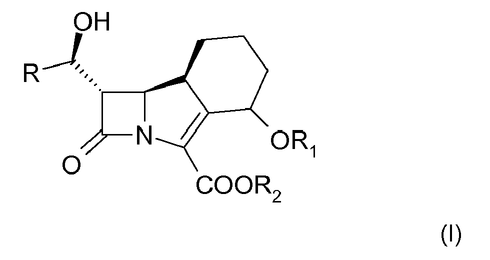 wherein
R represents a hydrogen atom or a saturated alkyl chain with 1 to 20 carbon atoms and the saturated alkyl chain may be straight (such as methyl, ethyl, n-propyl, n-butyl) or branched in any position (such as isopropyl, s-butyl, isobutyl, isoamyl, tert-butyl);
R¹ represents:
- a hydrogen atom,
- a saturated alkyl chain with 1 to 20 carbon atoms and the saturated alkyl chain may be straight (such as methyl, ethyl, n-propyl, n-butyl) or branched in any position (such as isopropyl, s-butyl, isobutyl, isoamyl, tert-butyl) and each chain member may be mono or disubstituted with substituents such as halo (such as fluoromethyl, tritluoromethyl, 2-chloroethyl), hydroxy (such as hydroxymethyl, 2-hydroxyethyl), (C_{I}-C₄)-alkyloxy (such as methoxymethyl, 2-methoxyethyl), mercapto and (C_{I}-C₄)-alkylmercapto (such as mercaptomethyl, 2-methylmercaptoethyl), (C_{I}-C₄)-alkanesulfonyl (such as methanesulfonylmethyl), amino, (C_{I}-C₄)-alkylamino and di(C_{I}-C₄)-alkylamino (such as 2-aminoethyl, 2-methylaminoethyl, 2-dimethylaminoethyl), alkyleneamino (such as 2-(1-piperidinyl)ethyl, 1-pyrrolidinylmethyl), guanidino (such as guanidinomethyl), unsubstituted N¹-mono, N³-mono, N¹,N³-di and N³,N³-di-(C_{I}-C₄)-formamidino (such as iminomethylaminomethyl, 2-(dimethylaminomethyleneamino)ethyl), aromatic or heteroaromatic five- or six-membered ring (such as phenyl, furyl, 2-pyridyl), (C_{I}-C₄)-alkyloxycarbonyl (such as carbethoxymethyl), cyano (such as 2-cyanoethyl), oxo (such as acetyl, propionyl, 2-oxopropyl),
- an unsaturated alkyl chain with 1 to 20 carbon atoms and the unsaturated alkyl chain may be straight with double bonds or triple bonds (such as vinyl, propenyl, allyl, ethinyl, propargyl) or branched in any position with double bonds or triple bonds (such as 2-propenyl) and each chain member may be mono or disubstituted with substituents such as halo, hydroxy, (C_{I}-C₄)-alkyloxy, thio and (C_{I}-C₄)-alkylthio, (C_{I}-C₄)-alkanesulfonyl, amino, (C_{I}-C₄)-alkylamino and di-(C_{I}-C₄)-alkylamino, aromatic or heteroaromatic five- or six-membered ring (such as phenyl, furyl, 2-pyridyl), (C_{I}-C₄)-alkyloxycarbonyl, cyano, oxo,
- a saturated or partly unsaturated cycloalkyl radical with 3 to 7 members (such as radicals from cyclopropyl to cycloheptyl, cyclohex-I-enyl) and the ring may comprise one or more oxygen, sulfur or nitrogen atoms (such as 2-tetrahydrofuranyl, 1-piperidinyl, 1-pyrrolidinyl) and each ring member may be mono or disubstituted with substituents such as halo, hydroxy, (C_{I}-C₄)-alkyloxy, thio and (C_{I}-C₄)-alkylthio), (C_{I}-C₄)-alkanesulfonyl, amino, (C_{I}-C₄)-alkylamino and di-(C_{I}-C₄)-alkylamino, (C_{I}-C₄)-alkyloxycarbonyl, cyano. oxo,
- an aromatic or heteroaromatic five- or six-membered ring (such as furyl, pyranyl),
- an alkanoyl (such as formyl, acetyl, benzoyl, ethoxycarbonyl, allyloxycarbonyl, pivaloyl), an alkenoyl (such as allylcarbonyl), an aroyl (such as p-nitrobenzoyl), an alkoxycarbonyl (such as t-buthoxycarbonyl), a haloalkoxy-carbonyl (such as 2,2,2-trichloroethoxycarbonyl, or 1,1,1-trichloro-2-methyl-2propoxycarbonyl), an aralkyloxycarbonyl (such as benzyloxycarbonyl or p-nitrobenzyloxycarbonyl), an alkenyloxycarbonyl (such as allyloxycarbonyl) mono, di or tri-(C_{I}-C₄)-alkylsilyl (such as trimethylsilyl, tert-butyldimethylsilyl) group,
- a (C_{I}-C₄)-alkanesulfonyl group (such as methanesulfonyl, ethanesulfonyl), a (C_{I}-C₄)-alkenesulfonyl group (such as allylsulfonyl), arylsulfonyl group (such as p-nitrobenzylsulfonyl);
R² represents:
- a hydrogen atom,
- an alkali metal,
- an earth alkali metal,
- the ammonium ion or a protonated form of mono, di or trisubstituted acyclic or cyclic aliphatic amine or a protonated form of some other nitrogen base,
- the quaternized ammonium ion,
- a (C_{I}-C₂₀)-alkyl (such as methylethyl, tert-butyl), (C₁-C₂₀)-alkenyl (such as allyl), substituted alkyl (such as (C_{I}-C₄)-alkoxyalkyl, (C_{I}-C₄)-alkylthioalkyl, phenetyl, 2,2,2-trichloroethyl, 2-oxo-5-methyl-1,3-dioxolene-4-yl)methyl, benzyl, p-methoxybenzyl, p-nitrobenzyl, o-nitrobenzyl, bis(methoxyphenyl)methyl, 3,4-dimethoxybenzyl, benzhydryl, trityl, 2-trimethylsilylethyl), substituted silyl (such as trimethylsilyl, tert-butyldimethylsilyl), phthalidyl etc., or
- a radical which may be presented in a following form 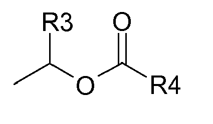 wherein R³ represents hydrogen or a lower alkyl with 1 to 4 carbon atoms, and
R⁴ represents hydrogen, alky, cycloalkyl, alkoxy, cycloalkoxy, cycloalkylalkyl, alkenyloxy, phenyl, 2-morpholinoethyl,
or a salt, ester or amide derivate of the compound of formula (I).

2. A pharmaceutical composition according to claim 1, wherein in formula (I), R represents a methyl group, or R¹ represents a methyl group or R and R¹ represent a methyl group.

3. A pharmaceutical composition according to claim 1 or 2, wherein the antibiotic is an beta-lactam antibiotic.

4. A pharmaceutical composition according to claim 3, wherein the beta-lactam antibiotic is selected from a group consisting of cephalosporins, penicillins, monobactams or carbapenems.

5. A pharmaceutical composition according to claim 4, wherein the cefalosporine is selected from a group consisting of ceftazidime, cefotaxime, cefepime, cefpirome, ceftobiprole or ceftaroline.

6. A pharmaceutical composition according to claim 4, wherein the penicilline is piperacilline.

7. A pharmaceutical composition according to any of the claims 1 to 6, wherein the pharmaceutical composition additionally comprises a pharmaceutically acceptable carrier.

8. A pharmaceutical composition according to any of the claims 1 to 7, wherein the pharmaceutical composition additionally comprises a pharmaceutically acceptable excipient.

9. A compound of the general formula (I) 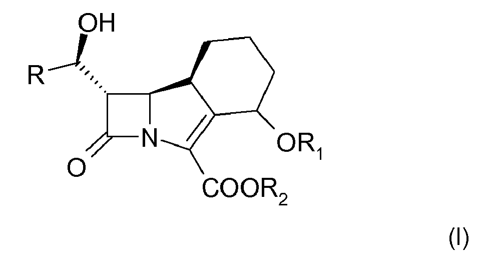 wherein
R represents a methyl group,
R1 represents a methyl group, and
R2 represents
- a (C_{I}-C₂₀)-alkyl (such as methylethyl, tert-butyl), (C₁-C₂₀)-alkenyl (such as allyl), substituted alkyl (such as (C_{I}-C₄)-alkoxyalkyl, (C_{I}-C₄)-alkylthioalkyl, phenetyl, 2,2,2-trichloroethyl, 2-oxo-5-methyl-1,3-dioxolene-4-yl)methyl, benzyl, p-methoxybenzyl, p-nitrobenzyl, o-nitrobenzyl, bis(methoxyphenyl)methyl, 3,4-dimethoxybenzyl, benzhydryl, trityl, 2-trimethylsilylethyl), substituted silyl (such as trimethylsilyl, tert-butyldimethylsilyl), phthalidyl etc., or
- a radical which may be presented in a following form 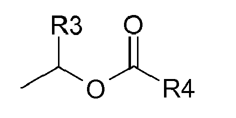
wherein R³ represents hydrogen or a lower alkyl with 1 to 4 carbon atoms, and
R⁴ represents hydrogen, alky, cycloalkyl, alkoxy, cycloalkoxy, cycloalkylalkyl, alkenyloxy, phenyl, 2-morpholinoethyl.

10. A therapeutically effective amount of one or more compounds of formula (I) as defined within claim 1 or a salt, ester or amide derivative thereof or a compound of formula (I) as defined in claim 9 for use as a broad-spectrum beta-lactamase inhibitor.

11. Use according to claim 10 wherein the beta-lactamase inhibitor is a beta-lactamase inhibitor of class A, C and D.

12. A therapeutically effective amount of one or more compounds of formula (I) as defined within claim 1 or a salt, ester or amide derivative thereof or a compound of formula (I) as defined in claim 9 for use as an antibiotic.

13. A pharmaceutical composition as claimed within any of the claims 1 to 8 or a compound as claimed in claim 9 for the treatment of an infection in humans or animals caused by bacteria.

14. Use of a therapeutically effective amount of the composition according to any of claims 1 to 8 or a compound according to claim 9 and at least one pharmaceutically acceptable excipient for the preparation of a medicament for treating an infection caused by bacteria, preferably wherein said medicament is to be administered to a patient in need thereof.

15. A method of treating an infection in humans or animals caused by bacteria comprising administering to a patient in need of such treating a therapeutically effective amount of the composition according to any of claims 1 to 8 or a compound according to claim 9 and at least one pharmaceutically acceptable excipient.
